# EUROPEAN PATENT APPLICATION

(11) **EP 4 721 689 A1**
(43) Date of publication of application: **08.04.2026**
(21) Application number: 25205211.3
(22) Date of filing: 29.09.2025
(51) Int. Cl.: A61B 34/10, A61B 34/20, A61B 34/30, A61B 34/00, A61B 90/00, B25J 9/16, B25J 19/00, A61B 34/37, G06N 3/02, G06N 20/00

(54) **TECHNIQUES FOR ESTIMATING DEFLECTION OF A SURGICAL ROBOTIC ARM**

(30) Priority: 30.09.2024 US 202463700968 P; 27.01.2025 US 202563749842 P
(71) Applicant: MAKO Surgical Corp., Weston, FL 33331 (US)
(72) Inventor: Talasaz, Ali, Plantation, 33325-2514 (US)
(74) Representative: Schott, Jakob Valentin

(57) **Abstract**

Surgical systems and methods involve a robotic arm comprising a plurality of links and joints and a surgical tool supported and moveable by the robotic arm and being configured to interact with an anatomy. Controller(s) coupled to the robotic arm are configured to estimate robotic arm deflection based on the tool interaction and/or characterize tool-anatomy interaction based on estimated arm deflection. To estimate the robotic arm deflection, the controller(s) input the pose of the surgical tool and the tool interaction force to a machine learning model. To characterize the interaction of the surgical tool with the anatomy, the controllers(s) input an operating parameter of the surgical tool and the estimated deflection into a machine learning model.

## Description

### CROSS-REFERENCE TO RELATED APPLICATIONS

The subject application claims priority to and all the benefits of United States Provisional Patent Application No. 63/700,968, filed September 30, 2024, and United States Provisional Patent Application No. 63/749,842 filed January 27, 2025, the entire contents of each of the aforementioned applications being hereby incorporated by reference.

### BACKGROUND

Robotic surgical systems that perform surgical procedures are well known and typically include a surgical robot and a surgical tool coupled to the surgical robot. For orthopedic surgery, the surgical tool is often controlled for removing tissue at the surgical site.

To ensure accurate operation of the surgical robot during a procedure, one or more robotic calibration processes are typically performed. The calibration processes involve identifying certain parameters of the surgical robot that may impact accuracy of the surgical robot.

There are various levels of robotic calibration to improve accuracy of the surgical robot. For example, level-1 calibration identifies the differences between actual and expected joint displacements. A higher order of calibration, i.e., level-2, involves kinematic calibration. Level-2 calibration is used to detect "geometric errors", such as those caused by link length, nonparallel axes, base misalignment, part tolerances, manufacturing, and assembly errors. Kinematic calibration addresses geometric errors by identifying the geometric parameters of the robotic kinematic structure to determine angle offsets and joint lengths, for example.

While level-1 and level-2 calibration are sufficient to address most errors in robotic surgery applications, they do not address non-geometric errors, which cause deflection of the robotic arm responsive to force(s) acting on the tool and/or robotic arm. Non-geometric errors can arise due to the payload, compliance/deflection, thermal deflection, and gear backlash. Non-geometric errors can affect the accuracy of the surgical tool. For example, in the context of robotic-assisted orthopedic surgery, a significant cutting force is applied during bone resection. The cutting force often depends on the bone density of the patient anatomy. In such cases, the joint/link compliance/deflection (non-geometric parameters) plays an important role in the cutting accuracy. For instance, a robot manipulator with a compliance of 40 microns/N can produce 2 mm of error for a typical cutting force of 50 N due solely to its compliance. However, level-1 and level-2 calibration are not adapted to address such errors. Moreover, the robot compliance is dependent on dynamic factors, such as pose and external forces, and is difficult to mathematically model.

Furthermore, another aspect that can affect the accuracy of the tool during surgery is the interaction of the tool with the anatomy. Often, the tool can experience excessive force in performing a surgical task, such as cutting. Conventional surgical systems typically obtain basic measurements related to the tool, such as force or temperature, and implement simple feedback loops to control the tool and reduce the tool's force on the anatomy. Conventional surgical robotic systems are not adapted to have a high-level of understanding of the tool's interaction with the anatomy. For example, such systems are not able to predict a level of workload or difficulty of the tool or why the tool is experiencing such excessive workload or difficulty (e.g., because of dense tissue interaction). This lack of deeper level understanding of the tool's interaction with the anatomy can result in inaccuracies and inefficiencies in the procedure. Moreover, the non-geometric errors, discussed above, can exacerbate the errors when the tool-anatomy force is excessive.

Accordingly, there is a need to develop technique(s) to estimate the effects of non-geometric errors on the accuracy of a robotically guided tool for surgical applications. There is also a need to develop technique(s) to classify/characterize a robotically guided tool's interaction with an anatomy.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided, comprising: a robotic arm comprising a plurality of links and joints; a surgical tool supported and moveable by the robotic arm and being configured to interact with an anatomy; a sensing system configured to detect an interaction force applied to the surgical tool based on an interaction of the surgical tool with the anatomy; and one or more controllers coupled to the robotic arm and the sensing system and being configured to: obtain, based on kinematic data from the robotic arm, a pose of the surgical tool; obtain, from the sensing system, the interaction force; input the pose of the surgical tool and the interaction force to a machine learning model; estimate a deflection of the robotic arm based on an output of the machine learning model; and optionally, control the robotic arm and/or the surgical tool based on the estimated deflection.

According to a second aspect, a surgical system is provided, comprising: a robotic arm comprising a plurality of links and joints; a surgical tool supported and moveable by the robotic arm and being configured to interact with an anatomy; a sensing system configured to detect: an interaction force applied to the surgical tool based on interaction of the surgical tool with the anatomy; a disturbance force applied to one or more of the links; and a user-applied force on the surgical tool; and one or more controllers coupled to the robotic arm and the sensing system and being configured to: obtain, based on kinematic data from the robotic arm, a pose of the surgical tool; obtain, from the sensing system, the interaction force, the disturbance force, and the user-applied force; input, to a machine learning model, the pose of the surgical tool, the interaction force, the disturbance force, and the user-applied force; and estimate a deflection of the robotic arm based on an output of the machine learning model.

According to a third aspect, a surgical system is provided, comprising: a robotic arm comprising a plurality of links and joints; a surgical tool supported and moveable by the robotic arm; a sensing system configured to detect a disturbance force applied to one or more of the links; and one or more controllers coupled to the robotic arm and the sensing system and being configured to: obtain, based on kinematic data from the robotic arm, a pose of the surgical tool; obtain, from the sensing system, the disturbance force; input the pose of the surgical tool and the disturbance force to a machine learning model; and estimate a deflection of the robotic arm based on an output of the machine learning model.

According to a fourth aspect, a surgical system is provided, comprising: a robotic arm comprising a plurality of links and joints; a surgical tool supported and moveable by the robotic arm; a sensing system configured to detect a user-applied force on the surgical tool; one or more controllers coupled to the robotic arm and the sensing system and being configured to: obtain, based on kinematic data from the robotic arm, a pose of the surgical tool; obtain, from the sensing system, the user-applied force; input the pose of the surgical tool and the user-applied force to a machine learning model; and estimate a deflection of the robotic arm based on an output of the machine learning model.

According to a fifth aspect, a surgical system is provided, comprising: a robotic arm comprising a plurality of links and joints; a surgical tool supported and moveable by the robotic arm and being configured to interact with an anatomy; a sensing system configured to detect an interaction force applied to the surgical tool based on an interaction of the surgical tool with the anatomy; and one or more controllers coupled to the robotic arm and the sensing system and being configured to: obtain, based on kinematic data from the robotic arm, a pose of the surgical tool; obtain, from the sensing system, the interaction force; input the pose of the surgical tool and the interaction force to a machine learning model; and estimate a deflection of the robotic arm based on an output of the machine learning model.

According to a sixth aspect, a surgical system is provided, comprising: a robotic arm comprising a plurality of links and joints; a surgical tool supported and moveable by the robotic arm and being configured to interact with an anatomy; a sensing system configured to detect one or more force applied to the surgical tool and/or the robotic arm; and one or more controllers coupled to the robotic arm and the sensing system and being configured to: detect presence of the one or more forces to trigger a deflection monitoring mode; and in the deflection monitoring mode, estimate a deflection of the robotic arm using a machine learning model.

According to a seventh aspect, a surgical system is provided, comprising: a robotic arm comprising a plurality of links and joints; a surgical tool supported and moveable by the robotic arm and being configured to interact with an anatomy; one or more controllers coupled to the robotic arm and being configured to: obtain an operating parameter of the surgical tool during an interaction of the surgical tool with the anatomy; estimate a deflection of the robotic arm resulting from the interaction of the surgical tool with the anatomy; input the operating parameter of the surgical tool and the estimated deflection into a machine learning model; and characterize the interaction of the surgical tool with the anatomy based on an output of the machine learning model.

According to an eighth aspect, a surgical system is provided, comprising: a robotic arm comprising a plurality of links and joints; a surgical tool supported and moveable by the robotic arm and being configured to interact with an anatomy; one or more controllers coupled to the robotic arm and being configured to: estimate a deflection of the robotic arm resulting from the interaction of the surgical tool with the anatomy; input the estimated deflection into a machine learning model; and characterize the interaction of the surgical tool with the anatomy based on an output of the machine learning model.

Also provided are a computer-implemented method of operating the surgical system of any of the aspects above; a non-transitory computer readable medium, or computer program product, comprising instructions, which when executed by one or more processors, are configured to execute the features of the one or more controllers described by any of the aspects above; a non-transitory computer readable medium, or computer program product, comprising instructions, which when executed by one or more processors, are configured to train or implement any of the machine learning models of any of the aspects above.

Any of the described aspects can be combined in whole, in or part.

Any of the described aspects can be combined in whole, or in part, with any of the following implementations. Any of the following implementations above can be combined in whole, or in part.

The controller(s) can control the robotic arm and/or the surgical tool based on the estimated deflection by being configured to: modify the tool path; modify the feed rate; adjust the commanded pose of the surgical tool; adjust a cutting speed of the tool; adjust a commanded pose of the surgical tool; halt movement of the surgical tool; and/or move the surgical tool away from the anatomy, or any combination thereof. The controller(s) can input an operating parameter (such as velocity) of the surgical tool and the estimated deflection into a second machine learning model and characterize the interaction of the surgical tool with the anatomy based on an output of the second machine learning model. The controller(s) can characterize a type of the anatomy, a workload of the surgical tool, a density of the bone, or combination thereof. The controller(s) can control the robotic arm and/or the surgical tool based on the characterized interaction by being configured to perform one or more of: modify a tool path of the surgical tool; modify a feed rate of the surgical tool; modify a cutting speed/direction of the surgical tool; adjust a commanded pose of the surgical tool; halt movement of the surgical tool; and/or move the surgical tool away from the anatomy. Monitoring of deflection can occur responsive to detection of certain system conditions, such as detection of force applied to the tool or manipulator. The machine learning model(s) can be a deep learning model, such as a deep learning model including at least two hidden layers. The estimated deflection can be a non-geometric deflection. The operating parameter of the surgical tool can be a velocity of the surgical tool, an average velocity of the surgical tool, an acceleration of the surgical tool, displacement of the surgical tool, a motor current of the surgical tool, or the like. The robotic arm can operate in a manual mode, a semi-automated mode, an automated mode, or a guided-manual mode.

### DESCRIPTION OF THE DRAWINGS

Advantages of the present disclosure will be readily appreciated as the same becomes better understood by reference to the following detailed description when considered in connection with the accompanying drawings.
Figure 1 is a perspective view of a robotic surgical system, according to one implementation.
Figure 2 is a block diagram of an example control system for controlling the robotic surgical system, according to one implementation.
Figure 3 is a functional block diagram of modules implemented by the control system, according to one implementation.
Figure 4 illustrates an example output of a boundary generator.
Figure 5 illustrates an example output of a path generator.
Figure 6 is a flowchart illustrating a technique or method for estimating deflection of a robotic arm resulting from tool-anatomy interaction by using a machine learning model, according to one implementation.
Figure 7 is a flowchart illustrating a technique or method for estimating a "total" deflection of a robotic arm resulting from one or more sources of forces by using a machine learning model, according to one implementation.
Figure 8 is a flowchart illustrating a technique or method for characterizing tool-anatomy interaction(s) using a machine learning model, according to one implementation.
Figure 9 is a chart illustrating an example output of the tool-anatomy interaction characterization of FIG. 8, wherein various interaction predictions are grouped into classifications of either bone, soft tissue, or free zone interaction.
Figure 10 is a chart illustrating another example output of the tool-anatomy interaction characterization of FIG. 8, wherein various interaction predictions are grouped into tissue density classifications.

### DETAILED DESCRIPTION

### I. Example System Overview

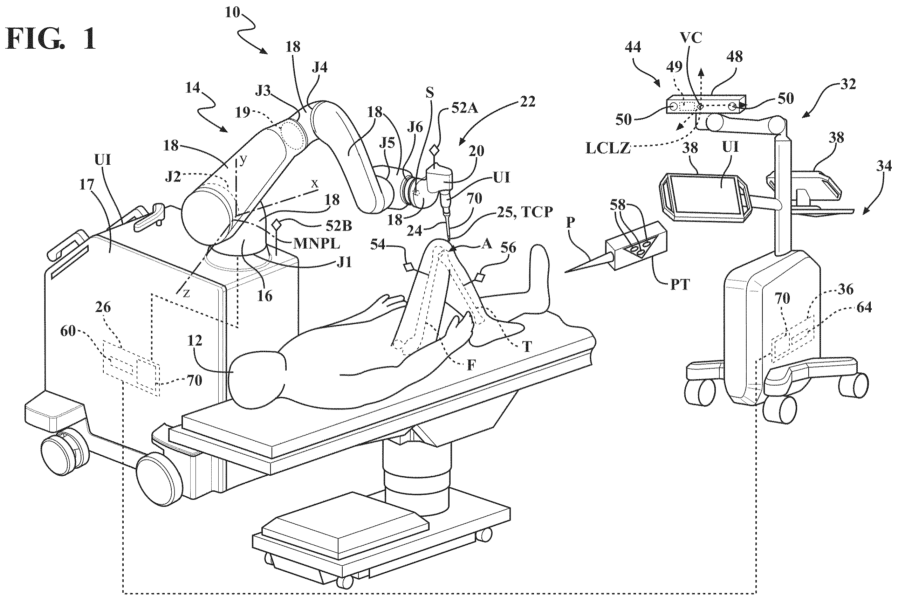

Referring to Figure 1, a surgical system, such as robotic surgical system 10 is illustrated. The system 10 is useful for treating a surgical site or anatomical volume (A) of a patient 12, such as treating bone or soft tissue. In Figure 1, the patient 12 is undergoing a surgical procedure. The anatomy in Figure 1 includes a femur F and a tibia T of the patient 12. The surgical procedure may involve tissue removal or other forms of treatment. Treatment may include cutting, coagulating, lesioning the tissue, other in-situ tissue treatments, or the like. In some examples, the surgical procedure involves partial or total knee or hip replacement surgery, shoulder replacement surgery, spine surgery, cranial surgery, or ankle surgery. In some examples, the system 10 is designed to cut away material to be replaced by surgical implants, such as hip and knee implants, including unicompartmental, bicompartmental, multicompartmental, or total knee implants. Some of these types of implants are shown in U.S. Patent Application Publication No. 2012/0330429, entitled, "Prosthetic Implant and Method of Implantation," the disclosure of which is hereby incorporated by reference. The system 10 and techniques disclosed herein may be utilized to perform other procedures, surgical or non-surgical, or may be utilized in industrial applications or other applications where robotic systems are utilized.

The system 10 includes a robotic manipulator 14. The manipulator 14 has a base 16 and plurality of links 18. A manipulator cart 17 supports the manipulator 14 such that the manipulator 14 is fixed to the manipulator cart 17. The links 18 collectively form the robotic arm(s) of the manipulator 14. The manipulator 14 may have a serial arm configuration (as shown in Figure 1), a parallel arm configuration, or any other suitable manipulator configuration. In other examples, more than one manipulator 14 may be utilized in a multiple arm configuration.

In the example shown in Figure 1, the manipulator 14 comprises a plurality of joints J and a plurality of joint encoders 19 located at the joints J for determining position data of the joints J. For simplicity, only one joint encoder 19 is illustrated in Figure 1, although other joint encoders 19 may be similarly illustrated. The manipulator 14 according to one example has six joints J1-J6 implementing at least six-degrees of freedom (DOF) for the manipulator 14. However, the manipulator 14 may have any number of degrees of freedom and may have any suitable number of joints J and may have redundant joints.

The manipulator 14 need not require joint encoders 19 but may alternatively, or additionally, utilize motor encoders present on motors at each joint J. Also, the manipulator 14 need not require rotary joints, but may alternatively, or additionally, utilize one or more prismatic joints. Any suitable combination of joint types is contemplated.

The base 16 of the manipulator 14 is a portion of the manipulator 14 that provides a fixed reference coordinate system for other components of the manipulator 14 or the system 10 in general. The origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the manipulator cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 17. In other examples, the manipulator 14 can be a hand-held manipulator where the base 16 is a base portion of a tool (e.g., a portion held free-hand by the user) and the tool tip is movable relative to the base portion. The base portion has a reference coordinate system that is tracked and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined.

The manipulator 14 and/or manipulator cart 17 house a manipulator controller 26, or other type of control unit. The manipulator controller 26 may comprise one or more computers, or any other suitable form of controller that directs the motion of the manipulator 14. The manipulator controller 26 may have a processing unit (CPU or GPU) and/or other processors and non-transitory memory. The manipulator controller 26 is loaded with software as described below. The processors could include one or more processors to control operation of the manipulator 14. The processors can be any type of microprocessor, multi-processor, and/or multi-core processing system. The manipulator controller 26 may additionally, or alternatively, comprise one or more microcontrollers, field programmable gate arrays, systems on a chip, discrete circuitry, and/or other suitable hardware, software, or firmware that is capable of conducting the functions described herein. The term processor is not intended to limit any embodiment to a single processor. The manipulator 14 may also comprise a user interface UI with one or more displays and/or input devices (e.g., push buttons, keyboard, mouse, microphone (voice-activation), gesture control devices, touchscreens, etc.).

A tool 20 couples to the manipulator 14 and is movable relative to the base 16 to interact with the anatomy in certain modes. The tool 20 is a physical and surgical tool and is, or forms part of, an end effector 22 supported by the manipulator 14 in certain implementations. The tool 20 may be grasped by the user. One arrangement of the manipulator 14 and the tool 20 can be like that described in U.S. Patent No. 9,119,655, entitled, "Surgical Manipulator Capable of Controlling a Surgical Instrument in Multiple Modes," the disclosure of which is hereby incorporated by reference. The manipulator 14 and the tool 20 may be arranged in alternative configurations. The tool 20 can be like that shown in U.S. Patent Application Publication No. 2014/0276949, filed on March 15, 2014, entitled, "End Effector of a Surgical Robotic Manipulator," hereby incorporated by reference.

The tool 20 can include an energy applicator 24 designed to contact and remove the tissue of the patient 12 at the surgical site. In one example, the energy applicator 24 is a bur 25. The bur 25 may be substantially spherical and comprise a spherical center, radius (r) and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc. The geometric feature may be considered to determine how to locate the tool 20 relative to the tissue at the surgical site to perform the desired treatment. In some of the embodiments described herein, a spherical bur having a tool center point (TCP) will be described for convenience and ease of illustration but is not intended to limit the tool 20 to any particular form. In other examples, the tool 20 does not include an energy applicator 24. For example, the tool 20 can be a slotted cut guide for a saw, a guide tube for receiving another tool, or the like.

The tool 20 may comprise a tool controller to control operation of the tool 20, such as to control power to the tool (e.g., to a rotary motor of the tool 20), control movement of the tool 20, control irrigation/aspiration of the tool 20, and/or the like. The tool controller may be in communication with the manipulator controller 26 or other components. The tool 20 may also comprise a user interface UI with one or more displays and/or input devices (e.g., push buttons, keyboard, mouse, microphone (voice-activation), gesture control devices, touchscreens, etc.). For example, one of the user input devices on the user interface UI of the tool 20 may be a tool input (e.g., switch or other form of user input device) that has first and second input states (see FIG. 1). The tool input can be actuated (e.g., pressed and held) by the user to be placed in the first input state and can be released to be placed in the second input state. The tool 20 may have a grip on which the tool input is located. In some versions, the tool input is a presence detector that detects the presence of a hand of the user, such as a momentary contact switch that switches between on/off states, a capacitive sensor, an optical sensor, or the like. The tool input is thus configured such that the first input state indicates that a user is actively engaging the tool 20 and the second input state indicates that the user has released the tool 20. The tool input may be a continuous activation device, i.e., inputs that must be continually actuated to allow motion of the tool 20 in the manual mode or the semi-autonomous mode, depending on which user input is actuated. For example, while the user is continually actuating the tool input, and the manual mode is enabled, the manipulator 14 will move in response to the input forces and torques applied by the user and the control system 60 will enforce a virtual object VO or virtual boundary 71 to protect the patient anatomy. When the tool input is released, input from the force/torque sensor S may be disabled such that the manipulator 14 no longer responds to the forces and torques applied by the user to the tool 20.

The manipulator controller 26 controls a state (position and/or orientation) of the tool 20 (e.g., the TCP) with respect to a coordinate system, such as the manipulator coordinate system MNPL. The manipulator controller 26 can control (linear or angular) velocity, acceleration, or other derivatives of motion of the tool 20. The tool center point (TCP), in one example, is a predetermined reference point defined at the energy applicator 24. The TCP has a known, or able to be calculated (i.e., not necessarily static), pose relative to other coordinate systems. The geometry of the energy applicator 24 is known in or defined relative to a TCP coordinate system. The TCP may be located at the spherical center of the bur 25 of the tool 20 such that only one point is tracked. The TCP may be defined in diverse ways depending on the configuration of the energy applicator 24. The manipulator 14 could employ the joint/motor encoders, or any other non-encoder position sensing method, to enable a pose of the TCP to be determined. The manipulator 14 may use joint measurements to determine TCP pose and/or could employ techniques to measure TCP pose directly. The control of the tool 20 is not limited to a center point. For example, any suitable primitives, meshes, etc., can be utilized to represent the tool 20.

The system 10 further includes a navigation system 32. One example of the navigation system 32 is described in U.S. Patent No. 9,008,757, filed on September 24, 2013, entitled, "Navigation System Including Optical and Non-Optical Sensors," hereby incorporated by reference. The navigation system 32 tracks movement of various objects. Such objects include, for example, the manipulator 14, the tool 20 and the anatomy, e.g., femur F and tibia T. The navigation system 32 tracks these objects to gather state information of each object with respect to a (navigation) localizer coordinate system LCLZ. Coordinates in the localizer coordinate system LCLZ may be transformed to the manipulator coordinate system MNPL, and/or vice-versa, using transformations.

The navigation system 32 includes a cart assembly 34 that houses a navigation controller 36, and/or other types of control units. A navigation user interface UI is in operative communication with the navigation controller 36. The navigation user interface includes one or more displays 38. The navigation system 32 is capable of displaying a graphical representation of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. However, the input devices may include any one or more of push buttons, a keyboard, a mouse, a microphone (voice-activation), gesture control devices, and the like.

The navigation system 32 also includes a navigation localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC.

The navigation system 32 includes one or more trackers. In one example, the trackers include a pointer tracker PT, one or more manipulator trackers 52A, 52B, a first patient tracker 54, and a second patient tracker 56. In the illustrated example of Figure 1, the manipulator tracker is coupled to the tool 20 (i.e., tracker 52A), the first patient tracker 54 is coupled to the femur F of the patient 12, and the second patient tracker 56 is coupled to the tibia T of the patient 12. In this example, the patient trackers 54, 56 are coupled to sections of bone. The pointer tracker PT is firmly affixed to a pointer P utilized for registering the anatomy to the localizer coordinate system LCLZ. The manipulator tracker 52A, 52B may be affixed to any suitable component of the manipulator 14, in addition to, or other than the tool 20, such as the base 16 (i.e., tracker 52B), or any one or more links 18 of the manipulator 14. The trackers 52A, 52B, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object with which it is associated.

Any one or more of the trackers may include active markers 58. The active markers 58 may include light emitting diodes (LEDs). Alternatively, the trackers 52A, 52B, 54, 56, PT may have passive markers, such as reflectors, which reflect light emitted from the camera unit 46. Other suitable markers not specifically described herein may be utilized.

The localizer 44 tracks the trackers 52A, 52B, 54, 56, PT to determine a state of each of the trackers 52A, 52B, 54, 56, PT, which correspond respectively to the state of the object respectively attached thereto. The localizer 44 may perform known triangulation techniques to determine the states of the trackers 52, 54, 56, PT, and associated objects. The localizer 44 provides the state of the trackers 52A, 52B, 54, 56, PT to the navigation controller 36. In one example, the navigation controller 36 determines and communicates the state the trackers 52A, 52B, 54, 56, PT to the manipulator controller 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 36 may comprise one or more computers, or any other suitable form of controller. Navigation controller 36 has a central processing unit (CPU) and/or other processors, non-transitory memory (not shown), and storage (not shown). The processors can be any type of processor, microprocessor, or multi-processor system. The navigation controller 36 is loaded with software. The software, for example, converts the signals received from the localizer 44 into data representative of the position and orientation of the objects being tracked. The navigation controller 36 may additionally, or alternatively, comprise one or more microcontrollers, field programmable gate arrays, systems on a chip, discrete circuitry, and/or other suitable hardware, software, or firmware that is capable of conducting the functions described herein. The term processor is not intended to limit any embodiment to a single processor.

Although one example of the navigation system 32 is shown that employs triangulation techniques to determine object states, the navigation system 32 may have any other suitable configuration for tracking the manipulator 14, tool 20, and/or the patient 12.

In another example, the navigation system 32 and/or localizer 44 are ultrasound-based. For example, the navigation system 32 may comprise an ultrasound imaging device coupled to the navigation controller 36. The ultrasound imaging device images any of the aforementioned objects, e.g., the manipulator 14, the tool 20, and/or the patient 12, and generates state signals to the navigation controller 36 based on the ultrasound images. The ultrasound images may be 2-D, 3-D, or a combination of both. The navigation controller 36 may process the images in near real-time to determine states of the objects. The ultrasound imaging device may have any suitable configuration and may be different than the camera unit 46 as shown in Figure 1.

In another example, the navigation system 32 and/or localizer 44 are radio frequency (RF)-based. For example, the navigation system 32 may comprise an RF transceiver coupled to the navigation controller 36. The manipulator 14, the tool 20, and/or the patient 12 may comprise RF emitters or transponders attached thereto. The RF emitters or transponders may be passive or actively energized. The RF transceiver transmits an RF tracking signal and generates state signals to the navigation controller 36 based on RF signals received from the RF emitters. The navigation controller 36 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52A, 52B, 54, 56, PT shown in Figure 1.

In yet another example, the navigation system 32 and/or localizer 44 are electromagnetically based. For example, the navigation system 32 may comprise an EM transceiver coupled to the navigation controller 36. The manipulator 14, the tool 20, and/or the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the navigation controller 36 based upon EM signals received from the trackers. The navigation controller 36 may analyze the received EM signals to associate relative states thereto. Again, such navigation system 32 examples may have structural configurations that are different than the navigation system 32 configuration shown in Figure 1.

The navigation system 32 may have any other suitable components or structure not specifically recited herein. Furthermore, any of the techniques, methods, and/or components described above with respect to the navigation system 32 shown may be implemented or provided for any of the other examples of the navigation system 32 described herein. For example, the navigation system 32 may utilize solely inertial tracking or any combination of tracking techniques, and may additionally or alternatively comprise, fiber optic-based tracking, machine-vision tracking, and the like.

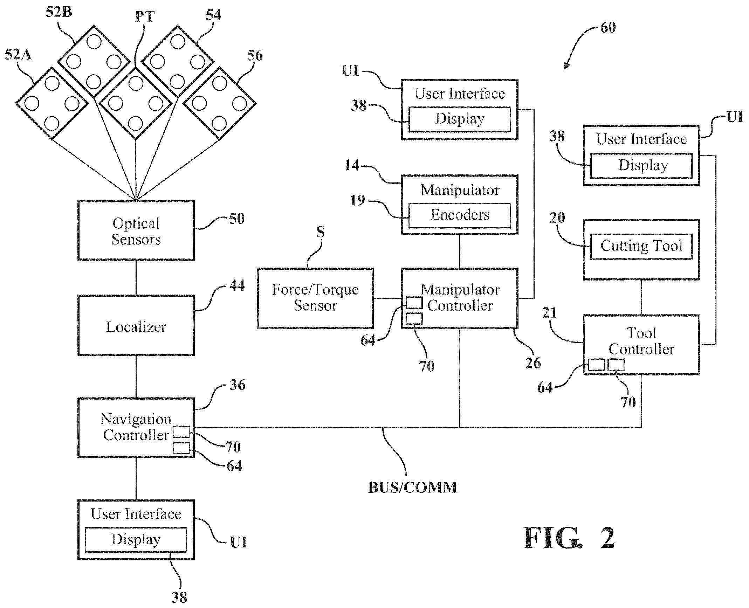

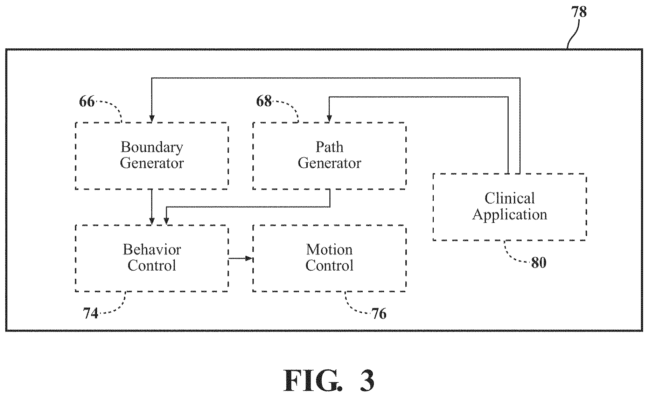

Referring to Figure 2, the system 10 includes a control system 60 that comprises, among other components, the manipulator controller 26, the navigation controller 36, and the tool controller 21. The control system 60 further includes one or more software programs and software modules shown in Figure 3. The software modules may be part of the program or programs that operate on the manipulator controller 26, navigation controller 36, tool controller 21, or any combination thereof, to process data to assist with control of the system 10. The software programs and/or modules include computer readable instructions stored in non-transitory memory 64 on the manipulator controller 26, navigation controller 36, tool controller 21, or a combination thereof, to be executed by one or more processors 70 of the controllers 21, 26, 36. The memory 64 may be any suitable configuration of memory, such as RAM, non-volatile memory, etc., and may be implemented locally or from a remote database. Additionally, software modules for prompting and/or communicating with the user may form part of the program or programs and may include instructions stored in memory 64 on the manipulator controller 26, navigation controller 36, tool controller 21, or any combination thereof. The user may interact with any of the input devices of the navigation user interface UI or other user interface UI to communicate with the software modules. The user interface software may run on a separate device from the manipulator controller 26, navigation controller 36, and/or tool controller 21.

The control system 60 may comprise any suitable configuration of input, output, and processing devices suitable for conducting the functions and methods described herein. The control system 60 may comprise the manipulator controller 26, the navigation controller 36, or the tool controller 21, or any combination thereof, or may comprise only one of these controllers. These controllers may communicate via a wired bus or communication network as shown in Figure 2, via wireless communication, or otherwise. The control system 60 may also be referred to as a controller. The control system 60 may comprise one or more microcontrollers, field programmable gate arrays, systems on a chip, discrete circuitry, sensors, displays, user interfaces, indicators, and/or other suitable hardware, software, or firmware that is capable of conducting the functions described herein.

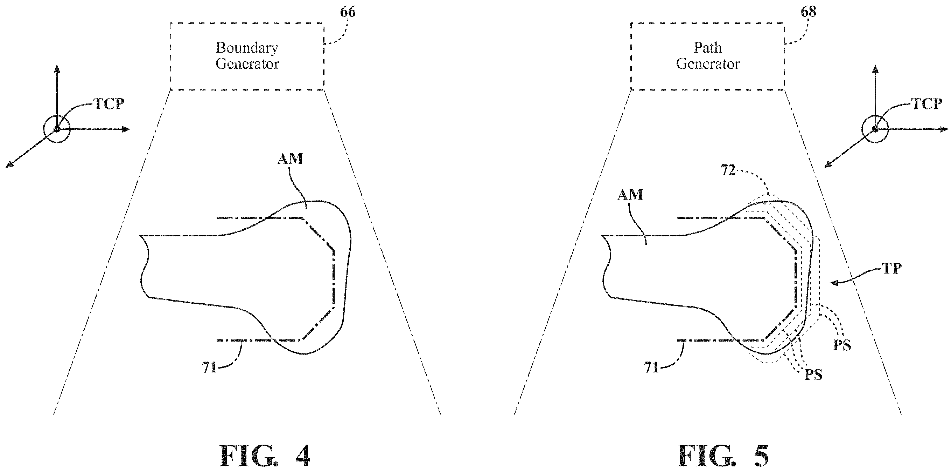

Referring to Figure 3, the software employed by the control system 60 includes a boundary generator 66. As shown in Figure 4, the boundary generator 66 is a software program or module that generates a virtual boundary 71 for constraining movement and/or operation of the tool 20. The virtual boundary 71 may be one-dimensional, two-dimensional, three-dimensional, and may comprise a point, line, axis, trajectory, plane, or other shapes, including complex geometric shapes. In some embodiments, the virtual boundary 71 is a surface defined by a triangle mesh. Such virtual boundaries 71 may also be referred to as virtual objects. The virtual boundaries 71 may be defined with respect to an anatomical model AM, such as a 3-D bone model. In the example of Figure 4, the virtual boundaries 71 are planar boundaries to delineate five planes for a total knee implant, and are associated with a 3-D model of the head of the femur F. The anatomical model AM is registered to the one or more patient trackers 54, 56 such that the virtual boundaries 71 become associated with the anatomical model AM. The virtual boundaries 71 may be implant-specific, e.g., defined based on a size, shape, volume, etc. of an implant and/or patient-specific, e.g., defined based on the patient's anatomy. The virtual boundaries 71 may be boundaries that are created pre-operatively, intra-operatively, or combinations thereof. In other words, the virtual boundaries 71 may be defined before the surgical procedure begins, during the surgical procedure (including during tissue removal), or combinations thereof. In any case, the control system 60 obtains the virtual boundaries 71 by storing/retrieving the virtual boundaries 71 in/from memory, obtaining the virtual boundaries 71 from memory, creating the virtual boundaries 71 pre-operatively, creating the virtual boundaries 71 intra-operatively, or the like.

The manipulator controller 26 and/or the navigation controller 36 track the state of the tool 20 relative to the virtual boundaries 71. In one example, the state of the TCP is measured relative to the virtual boundaries 71 for purposes of determining haptic forces to be applied to a virtual rigid body model via a virtual simulation 88 so that the tool 20 remains in a desired positional relationship to the virtual boundaries 71 (e.g., not moved beyond them). The results of the virtual simulation 88 are commanded to the manipulator 14. The control system 60 controls/positions the manipulator 14 in a manner that emulates the way a physical handpiece would respond in the presence of physical boundaries/barriers. The boundary generator 66 may be implemented on the manipulator controller 26. Alternatively, the boundary generator 66 may be implemented on other components, such as the navigation controller 36.

Referring to Figures 3 and 5, a path generator 68 is another software program or module run by the control system 60. In one example, the path generator 68 is run by the manipulator controller 26. The path generator 68 generates a tool path TP for the tool 20 to traverse. The tool path TP may comprise a plurality of path segments PS, or may comprise a single path segment PS. The path segments PS may be straight segments, curved segments, combinations thereof, or the like. The tool path TP may be defined with respect to the manipulator 14 coordinate system MNPL, localizer coordinate system LCLZ, coordinate system of the tool 20, coordinate system of the anatomy, or any combination thereof. The tool path TP can be virtually attached to the coordinate system of the respective object such that if the object were to move, the tool path TP will correspondingly move. The tool path TP may be implant-specific, e.g., defined based on a size, shape, volume, etc. of an implant and/or patient-specific, e.g., defined based on the patient's anatomy. The tool path TP can be associated with a virtual model of the anatomy and the virtual model and tool path can be registered to the anatomy using the navigation system 32. The control system 60 can generate or obtain the tool path TP by storing/retrieving the tool path TP in/from memory, creating the tool path TP pre-operatively, creating the tool path TP intra-operatively, or the like. The tool path TP may have any 3D shape, or combinations of shapes, such as circular, helical/corkscrew, linear, curvilinear, combinations thereof, and the like.

In one implementation, the tool path TP is defined as a guidance or alignment path. In one example, the tool path TP is for guiding the tool 20 to move to a location that positions the tool 20 for a start of the surgical procedure, or step. For instance, if the tool 20 is a saw blade, the tool path TP may be configured to guide the saw blade to align to a cut plane associated with the anatomy. If the tool 20 is a cutting bur, the tool path TP may be configured to guide the cutting bur to a starting point in preparation for automated cutting. A lead-in path could be virtually connected from the starting point to another cutting path for removal of tissue. The tool path TP may also enable the tool 20 to move along a predefined path of motion for purposes of registering components of the manipulator 14 to the navigation system 32. The tool path TP can be registered to the anatomy using the navigation system 32 such that the tool path TP is virtually fixed to the anatomy. This way, the tool path TP location in space will automatically be updated to account for any movement of the anatomy.

In another implementation, as shown in FIG. 5, the tool path TP is defined as a tissue removal path. One example of the tissue removal path described herein comprises a milling path 72. The term "milling path" refers to the path of the tool 20 in the vicinity of the target site for milling the anatomy and is not intended to require that the tool 20 be operably milling the anatomy throughout the entire duration of the path. For instance, as will be understood in further detail below, the milling path 72 may comprise sections or segments where the tool 20 transitions from one location to another without milling. Additionally, other forms of tissue removal along the milling path 72 may be employed, such as tissue ablation, and the like. The milling path 72 may be a predefined path that is created pre-operatively, intra-operatively, or combinations thereof. In other words, the milling path 72 may be defined before the surgical procedure begins, during the surgical procedure (including during tissue removal), or combinations thereof.

One example of a system and method for generating the virtual boundaries 71 and/or the milling path 72 is described in U.S. Patent No. 9,119,655, entitled, "Surgical Manipulator Capable of Controlling a Surgical Instrument in Multiple Modes," the disclosure of which is hereby incorporated by reference. In some examples, the virtual boundaries 71 and/or tool paths TP may be generated offline rather than on the manipulator controller 26 or navigation controller 36. Thereafter, the virtual boundaries 71 and/or tool paths TP may be utilized at runtime by the manipulator controller 26.

Referring to Figure 3, two additional software programs or modules run on the manipulator controller 26 and/or the navigation controller 36. One software module performs behavior control 74. Behavior control 74 is the process of computing data that indicates the next commanded pose and/or orientation (e.g., pose) for the tool 20. In some cases, only the position of the TCP is output from the behavior control 74, while in other cases, the position and orientation of the tool 20 is output. Output from the boundary generator 66, the path generator 68, and a force/torque sensor S may feed as inputs into the behavior control 74 to determine the next commanded pose and/or orientation for the tool 20. The behavior control 74 may process these inputs, along with one or more virtual constraints described further below, to determine the commanded pose.

The second software module performs motion control 76. One aspect of motion control is the control of the manipulator 14. The motion control 76 receives data defining the next commanded pose from the behavior control 74. Based on these data, the motion control 76 determines the next position of the joint angles of the joints J of the manipulator 14 (e.g., via inverse kinematics and Jacobian calculators) so that the manipulator 14 is able to position the tool 20 as commanded by the behavior control 74, e.g., at the commanded pose. In other words, the motion control 76 processes the commanded pose, which may be defined in Cartesian space, into joint angles of the manipulator 14, so that the manipulator controller 26 can command the joint motors accordingly, to move the joints J of the manipulator 14 to commanded joint angles corresponding to the commanded pose of the tool 20. In one version, the motion control 76 regulates the joint angle of each joint J and continually adjusts the torque that each joint motor outputs to, as closely as possible, ensure that the joint motor drives the associated joint J to the commanded joint angle.

The boundary generator 66, path generator 68, behavior control 74, and motion control 76 may be sub-sets of a software program 78. Alternatively, each may be software programs that operate separately and/or independently in any combination thereof. The term "software program" is used herein to describe the computer-executable instructions that are configured to conduct the various capabilities of the technical solutions described. For simplicity, the term "software program" is intended to encompass, at least, any one or more of the boundary generator 66, path generator 68, behavior control 74, and/or motion control 76. The software program 78 can be implemented on the manipulator controller 26, navigation controller 36, or any combination thereof, or may be implemented in any suitable manner by the control system 60.

A clinical application 80 may be provided to manage user interaction. The clinical application 80 manages many aspects of user interaction and coordinates the surgical workflow, including pre-operative planning, implant placement, registration, bone preparation visualization, and post-operative evaluation of implant fit, etc. The clinical application 80 is configured to output to the displays 38. The clinical application 80 may run on its own separate processor or may run alongside the navigation controller 36. In one example, the clinical application 80 interfaces with the boundary generator 66 and/or path generator 68 after implant placement is set by the user, and then sends the virtual boundary 71 and/or tool path TP returned by the boundary generator 66 and/or path generator 68 to the manipulator controller 26 for execution. Manipulator controller 26 executes the tool path TP as described herein. The manipulator controller 26 may additionally create certain segments (e.g., lead-in segments) when starting or resuming machining to smoothly get back to the generated tool path TP. The manipulator controller 26 may also process the virtual boundaries 71 to generate corresponding virtual constraints as described further below.

The system 10 may operate in a manual mode, such as described in U.S. Patent No. 9,119,655, incorporated herein by reference. Here, the user manually directs, and the manipulator 14 executes movement of the tool 20 and its energy applicator 24 at the surgical site. The user physically contacts the tool 20 to cause movement of the tool 20 in the manual mode. In one version, the manipulator 14 monitors forces and torques placed on the tool 20 by the user to position the tool 20. For example, the manipulator 14 may comprise the force/torque sensor S that detects the forces and torques applied by the user and generates corresponding input utilized by the control system 60 (e.g., one or more corresponding input/output signals). In some implementations, the user may be required to continually grasp a trigger or switch on the end effector to enable the force/torque sensor S that detects the forces and torques applied by the user.

The force/torque sensor S may comprise a 6-DOF force/torque transducer. The manipulator controller 26 and/or the navigation controller 36 receives the input (e.g., signals) from the force/torque sensor S. In response to the user-applied forces and torques, the manipulator 14 moves the tool 20 in a manner that emulates the movement that would have occurred based on the forces and torques applied by the user. This can enable the manipulator 14 to operate using an admittance-based system. Movement of the tool 20 in the manual mode may also be constrained in relation to the virtual boundaries 71 generated by the boundary generator 66. In some versions, measurements taken by the force/torque sensor S are transformed from a force/torque coordinate system FT of the force/torque sensor S to another coordinate system, such as a virtual mass coordinate system VM in which the virtual simulation 88 is carried out on the virtual rigid body model of the tool 20 so that the forces and torques can be virtually applied to the virtual rigid body in the virtual simulation 88 to ultimately determine how those forces and torques (among other inputs) would affect movement of the virtual rigid body, as described below. In another example, the manipulator 14 can operate using an impedance control system. In such examples, the user manipulates the tool 20 to the target and controls its position. Then, the manipulator controller 26 can generate a reactive force based on the interaction of the tool 20 with a physical or virtual object. The techniques described herein can be utilized with any type of impedance or admittance-controlled manipulator 14.

The system 10 may also operate in a semi-autonomous or automated mode in which the manipulator 14 moves the tool 20 along the milling path 72 (e.g., the active joints J of the manipulator 14 operate to move the tool 20 without requiring force/torque on the tool 20 from the user). An example of operation in the automated mode is also described in U.S. Patent No. 9,119,655, incorporated herein by reference. In some embodiments, when the manipulator 14 operates in the automated mode, the manipulator 14 is capable of moving the tool 20 free of user applied forces. In other words, the user does not need to physically contact the tool 20 to move the tool 20. Instead, the user may use some form of remote control to control starting and stopping of movement. For example, the user may hold down a button of the remote control to start movement of the tool 20 and release the button to stop movement of the tool 20.

The system 10 may also operate in a guided-manual mode, as described in U.S Patent Application Publication No. US 2020/0281676 A1, entitled "Systems and Methods for Controlling Movement of a Surgical Tool Along a Predefined Path", the contents of which are hereby incorporated by reference in their entirety. In the guided-manual mode, the user applies forces/torques to the force/torque sensor S and the applied forces/torques are utilized to determine how far to advance the tool 20 along the tool path TP. In the guided-manual mode, the tool 20 is constrained to the tool path TP in 2DOF normal to the tool path, but unconstrained in 1DOF tangential to the tool path TP. In effect, this enables the tool 20 to freely move along the tool path TP based on manual input, but the constraints guide the user by restricting the manual movement of the tool 20 to be along the tool path.

### II. Techniques For Estimating Deflection Of A Surgical Robotic Arm

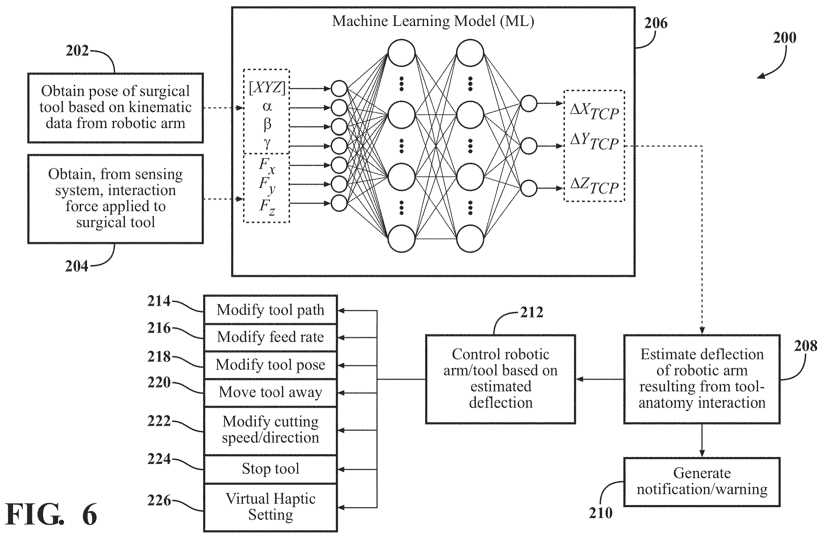

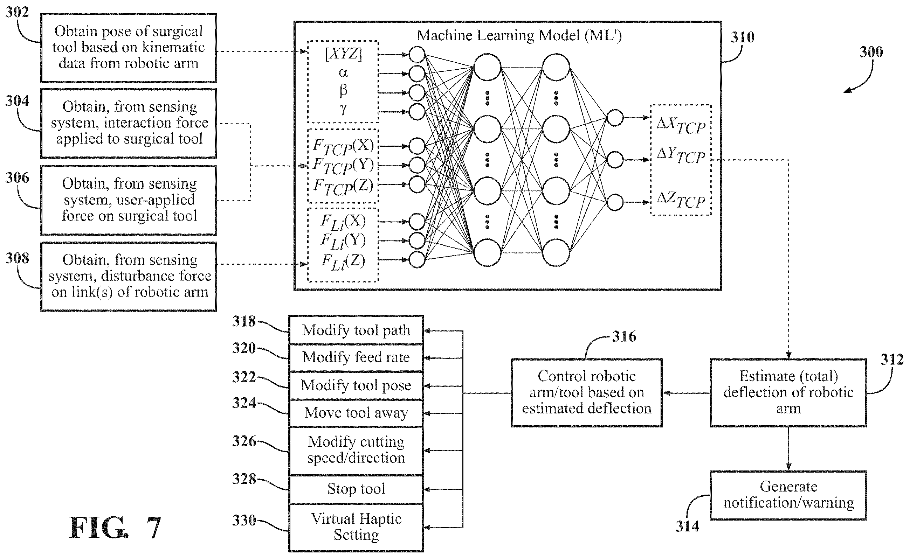

Referring to Figures 6 and 7, described herein are various techniques for estimating deflection of the manipulator 14. The techniques described herein can be implemented by any of the described control system(s), controller(s), and/or processors including but not limited to the control system 60, manipulator controller 26, tool controller 21, navigation controller 36, or any combination thereof. For simplicity, the one or more devices will be referred to herein as "the controller(s)." In some instances, any of the data described herein can be sourced from or transmitted to a remote server.

The controller(s) are configured to estimate robotic arm deflection based on the interaction between the tool 20 and the tissue of the anatomy A. The controller(s) can also characterize tool-anatomy interaction based on estimated arm deflection. To estimate the robotic arm deflection, the controller(s) input the pose of the tool 20 and the tool interaction force to a machine learning model. To characterize the interaction of the surgical tool with the anatomy, the controllers(s) input an operating parameter of the surgical tool 20 and the estimated deflection into a machine learning model. The controller(s) can optionally control the manipulator 14 based on any of the described arm deflection estimates and/or tool- anatomy interaction characterizations.

In turn, the techniques described herein advantageously provide a calibration and/or compensation for the effects of non-geometric errors on the accuracy of the manipulator-guided surgical tool 20 for surgical applications. Unlike prior techniques, the calibration/compensation can be dynamically computed and implemented in real-time. The accuracy of the tool 20 in cutting/resecting the anatomy will be increased thereby improving clinical outcomes and reducing surgical operating time needed to correct cutting inaccuracies resulting from non-geometric errors.

Moreover, unlike the prior techniques, the proposed techniques are limited to a calibration. In one case, it can be assumed that the manipulator 14 has been already calibrated and the techniques provide an "on the fly" control process during robotic surgical cutting rather than a one-time calibration scheme. The machine learning networks described herein can be trained for the real cutting forces in all XYZ directions for the entire arm workspace. In some instances, the techniques utilize a non-linear deep neural network for the compliance model. The non-linear deep neural network can model the compliance of the arm itself without any presumption on the compliance model. Unlike the prior techniques, the attributes (inputs) of the machine learning model can be the measured forces (like the force measured by the force/torque sensor) and the cartesian robot poses at the TCP (rather than simply using joint angle inputs). The machine learning networks described herein may only need to be trained once on one particular robot of the type and the results can be applied to all other robots from the same type. The techniques eliminate the need for training each individual robot manipulator.

### A. Sensing System and Detection of Force(s)

Various forces can be utilized in the deflection estimation and tissue-interaction characterization techniques described herein. The forces include, but are not limited to: an interaction force, a disturbance force, and/or a user-applied force. These various forces can be detected by any one or more components of a "sensing system." The components of the sensing system can be located at the manipulator 14, surgical tool 20, navigation system 32, combinations thereof, or located in any other suitable location. One component of the sensing system can include the force/torque sensor S, described above, which can detect external forces/torques applied to the surgical tool 20. In other examples, the sensing system could employ force observers that can estimate any of the described forces using computational methods, with or without using sensors. The sensing system can include other components such as the joint encoders 19 or other position sensors of the various joints J of the robotic manipulator 14. In some cases, actual joint positions/torques can be compared with expected joint positions/torques. The sensing system can also utilize current sensor(s) to detect the electrical current draw of the various joint J motors/actuators of the manipulator 14. The one or more joints can include force/torque sensors for measuring joint torque/force. In some cases, pressure sensors can be applied to the surface(s) of one or more link(s) of the manipulator 14 to detect external force placed on the robotic arm. The navigation system 32 can be used as part of the sensing system, for example, to determine the pose of the manipulator 14 or surgical tool 20 (from the trackers 52A, 52B.) The pose determined by the navigation system 32 can be compared with the pose determined from kinematic data of the manipulator to make comparisons or inferences about applied force. The sensing system can utilize tool sensors to detect operational parameters of the tool 20, such as motor current, resistance/impedance, cutting speed, velocity, or the like. The sensing system can include inertial sensors (such as accelerometers, gyroscopes, magnetometers, or the like) coupled to any component of the manipulator 14 and/or tool 20. The sensing system can utilize any one or more of these methods of measuring applied force on the manipulator 14. Equivalents of these sensing techniques are also contemplated.

One force that can be detected by the sensing system is the interaction force. In one implementation, the interaction force is a force applied to the surgical tool 20 based on an interaction of the surgical tool 20 with the anatomy or patient tissue. The type of interaction force will depend on the type of tool 20 and operation of the tool 20. For example, if the tool 20 is a tissue removal tool, such as a rotary cutter or saw blade, the interaction force can be a tissue removal (or cutting) force resulting from the tool 20 removing tissue. If the tool 20 is a probing tool, the interaction force can be a pressure resulting from the tool 20 pressing into the tissue. If the tool 20 is an impactor tool, the interaction force can be an impaction force resulting from the tool 20 impacting into the anatomy, and so on. In some instances, the interaction force can be a result of the tool 20 interacting with an obstacle or unintended object, such as a retractor, incision opening, or the like. The interaction force can be responsive to haptic feedback provided to the tool 20 based on the tool 20 interaction with a virtual object or boundary associated with the anatomy A. The virtual object can create a reaction force against the user motion. The interaction force may be any number of force(s) and/or torque(s) in any number of degrees of freedom. In one example, the interaction force can be measured with respect to the TCP of the tool 20. One component of the sensing system that may be well-suited for detecting the interaction force is the force/torque sensor S, which can be located between the tool 20 and the distal link of the manipulator 14. Additionally, or alternatively, other sensing methods can be utilized to detect the interaction force, such as sensing tool operating parameters, strain gauges on the tool 20 or energy applicator, or the like.

Another force that can be detected by the sensing system is the user-applied force applied to the surgical tool 20. Here, the user is any human operator (staff/surgeon) that interacts physically with the manipulator 14, or tool 20. For example, the operator may grasp the tool 20 and apply force to move the tool 20. This movement can be to perform cutting/manipulation of the anatomy with the tool 20 or can be movement for any other purpose. The type of user-applied force may depend on the type of tool 20 and surgical step/operation to be performed. For example, if the tool 20 is a tissue removal tool, such as a rotary cutter, the user-applied force may result from the user applying force to move the tool 20 along a predetermined or free tool path. If the tool 20 is a drilling tool, the user-applied force may result from the user applying force to advance the tool 20 along a trajectory, and so on. The user-applied force may be any number of force(s) and/or torque(s) in any number of degrees of freedom. In one example, the user-applied force can be measured with respect to the TCP of the tool 20. The sensing system can utilize the force/torque sensor S to detect the user-applied force. Additionally, or alternatively, other sensing methods can be utilized to detect the user-applied force, such as a load cell on the tool 20 itself, or any other sensing method described.

The disturbance force is another force that can be detected by the sensing system. The disturbance force is one applied to one or more of the links 18 of the manipulator 14. The disturbance force can be a contact force from a known or unknown source, which can cause deflection of the robotic arm links. The disturbance can result from sources. For example, the disturbance force can be due to the interaction force, the user-applied force on the tool 20, a user-applied force on the robotic arm, a collision with the robotic arm, unexpected payload, gravity, backdrive forces, or the like. The disturbance force may be any number of force(s) and/or torque(s) in any number of degrees of freedom. The sensing system can utilize joint motor current or torque sensors in the joints J to detect the disturbance force. Additionally, or alternatively, other sensing methods can be utilized to detect the disturbance force, such as comparing expected to actual joint torque, or using a pressure sensor on the link(s) 18, and the like.

The techniques described herein can utilize any one or more, or all of, the described forces, if available and detected. However, not all forces need to be utilized, or even detected. For instance, the techniques may utilize the interaction force and user-applied force, but not the disturbance force. The forces can be detected at the same time or at different times. The forces may be related to the same event or may result from different events. The forces can be the same, partially related, or completely independent from each other. For instance, the interaction force and the user-applied force can be related or unrelated depending on the conditions. For automated or semi-automated operation of the manipulator 14, there may be an interaction force due to the tool 20 moving along a path, but no user-applied force since the tool 20 is being moved automatically by the manipulator 14. For manual operation of the manipulator 14, there may be an interaction force that is substantially equivalent to the user-applied force, due to the user manually pushing the tool 20 into the tissue.

The controller(s) are configured to map any of the described forces into the manipulator coordinate system MNPL. For example, the force(s) can be mapped to the base 16 of the manipulator 14 to provide a common reference. Mapping to the base 16 can enable comparisons to the kinematic data of the manipulator 14 and TCP pose relative to the base 16 during tracked movement of the tool 20 by the navigation system 32. Having described the various forces that can be detected, the subsequent sections will now describe the deflection estimation and tissue-interaction characterization techniques that utilize some or all of these forces to make predictions or control the manipulator 14.

### B. Arm Deflection Estimation Based On Tool-Anatomy Interaction

According to one implementation, as shown in FIG. 6, a technique/method 200 implemented by the controller(s) is provided for estimating the deflection of the manipulator 14 responsive to interaction of the tool 20 with the anatomy A. This estimated deflection is a non-geometric error. Not all steps shown in FIG. 6 are required and some steps may be optional.

At 202, the controller(s) obtain the pose of the surgical tool 20 based on kinematic data from the manipulator 14. The kinematic data can be derived from the joint J encoder measurements and kinematic model defining the links 18 and joints J of the manipulator 14. Additionally, or alternatively, the pose of the surgical tool 20 can be obtained by the navigation system 32 tracking the manipulator 14 and/or surgical tool 20 using the trackers 52a, 52b, for example. Kinematic data and tracking data can be fused or compared as needed to obtain the tool 20 pose. The pose of the surgical tool 20 may specifically be a pose of the TCP of the tool 20. The TCP pose can be mapped to the base 16 of the manipulator 14. The pose of the tool 20 can be defined by its position and/or orientation defined by components in six degrees-of-freedom or the pose of the tool 20 can be uniquely defined by its distance to the robot base coordinate system and its orientation. The pose of the tool 20 can be obtained at a time step, T. The pose of the tool 20 can be obtained at discrete times or continuously over a period of time.

At 204, the controller(s) obtain the interaction force applied to the surgical tool 20 responsive to interaction of the surgical tool 20 with the anatomy or patient tissue. The interaction force is derived based on measurements from the sensing system. As described above, the sensing system can obtain such measurements from various sources. In one implementation, the measurements are provided by the force/torque sensor S coupled between the manipulator 14 and the tool 20 and the interaction force is more specifically a cutting force responsive to the tool 20 cutting bone. The interaction force may be defined relative to the TCP of the tool 20 and defined by three axial force components, or force/torque components in six degrees-of-freedom. The interaction force can be obtained at a time step, T, e.g., the same time the pose of the tool 20 is obtained at step 202. The interaction force can be obtained at discrete times or monitored over a period of time. The period of time can be triggered in response to certain conditions, such as initial detection of the interaction force.

At 206, the controller(s) input the pose of the surgical tool 20 and the interaction force to a machine learning model ML. The machine learning model ML can be utilized for "on the fly" non-geometric error compensation during surgery.

In one example, the machine learning model ML is a deep learning model that includes at least two hidden layers. The machine learning model can be any one or more types of models, such as: a neural networks, convolutional neural network, deterministic policy gradient (DPG) or deep DPG, deep Q-learning, recurrent neural network, generative adversarial network, supervised machine learning models, Regression algorithms, classification algorithms, Naive Bayes classifiers, random forest algorithms, unsupervised machine learning models, K-means clustering, hierarchical clustering, probabilistic clustering, reinforcement learning, NLP models, transformer networks, autoencoders, actor-critic networks, or the like.

The machine learning model ML takes values from the pose of the surgical tool 20 and the interaction force at an input layer having an appropriate number of input nodes/neurons for the number of input values. The hidden layers learn how much the robotic arm deflection occurs for different interaction forces at different arm configurations during tool interaction with the anatomy A. The hidden layers iteratively become more accurate at making predictions based on the input data, and potentially the output data, if supervised.

The machine learning model ML can be trained or pre-trained in various manners. The hidden layers can be trained online or offline on predetermined values of tool pose and interaction forces. For example, the machine learning model ML can be trained on a manipulator 14 that is already calibrated using level-1 and level-2 kinematic (geometric) calibration to isolate and learn non-linear compliance of the robotic arm for various arm configurations. In some cases, during training, a varying payload can be attached to the manipulator 14. A laser tracker can be utilized to train the network. Laser tracker is an accurate measurement device, up to 16 microns, which can determine the position of Spherically Mounted Retroreflectors (SMRs) held against the object to be measured. A special end effector with adjustable weights (up to 10 Kg) can be utilized with multiple SMRs to collect XYZ position data for different arm poses covering the workspace of the manipulator 14. This helps to better exercise the joints and collect more points for different arm poses under a variant load force mimicking cutting force during surgery. Additionally, or alternatively, the hidden layers can be trained online based on values of tool pose and interaction forces acquired in real-time during surgery.

The output of the machine learning model ML may be an output layer comprising any suitable number of nodes. For example, the output layer may include three nodes, each node predicting the estimated deflection of the manipulator 14 for one axial degree of freedom (x, y, z). The output can be characterized as the x, y, z error displacement of the TCP position relative to its commanded/controlled position at the time step T, the time at which the interaction force and tool pose were acquired. The output can also predict the deflection in the orientation (pitch, roll, yaw) of the tool 20 due to non-geometric errors.

At 208, the controller(s) utilize the output values of the machine learning model ML to estimate the deflection of the manipulator 14 and/or surgical tool 20 resulting from the interaction of the tool 20 with the anatomy A. In some instances, a deflection threshold can be utilized to filter negligible deflection estimates and acknowledge deflection estimates that are above the threshold. Additionally, or alternatively, a time threshold can be utilized to filter deflections estimates of short duration and acknowledge deflection estimates of duration above the time threshold.

The controller(s) can utilize the machine learning model ML to output the estimated deflection for any number of time-steps during runtime of the manipulator 14. For example, the estimated deflection values can be generated for every time step. In some cases, to reduce computational resources, the controller(s) can trigger a deflection monitoring mode during which the machine learning model ML is utilized for making deflection predictions. The deflection monitoring mode may be triggered based on certain conditions related to the tool 20, the anatomy A, or the surgical procedure. For example, the deflection monitoring mode can be triggered in response to detecting any interaction force. In other example, the deflection monitoring mode can be triggered based on tracked data from the navigation system 32, e.g., indicating that the tool 20 is within a threshold distance to the anatomy A, or contacting the anatomy A. In another example, the deflection monitoring mode is triggered in response to the user pressing trigger to operate the tool 20 or in response to the force/torque sensor S generating readings.

Using the estimated deflection (optionally filtered by thresholding), the controller(s) can perform various tasks. Any of the following tasks can be performed for a subsequent time step T + 1 and can persist thereafter. Any of the tasks described herein can be combined in part, or in whole, and can be executed simultaneously or at different times.

In one example, at 210, the controller(s) can generate a warning/notification related to the deflection. For instance, the warning/notification can be immediately presented on the display 38 of the navigation system 32 to communicate to the operator a message, such as "Warning -Loss of Robotic Accuracy", or the like. Additionally, or alternatively, the warning can be conveyed through the manipulator 14 to suggest that the applied force is excessive. For instance, the controller(s) can provide a vibratory feedback to the user through the surgical tool 20 or can illuminate an indicator on the manipulator 14 a certain color, such as red. Other types of audio/visual notifications can also be communicated to the operator.

Additionally, or alternatively, at 212, the controller(s) can control the manipulator 14 and/or tool 20 based on the estimated deflection. There are various examples for how the controller(s) can control the manipulator 14 and/or tool 20 based on the estimated deflection. The control can be performed to improve accuracy of the tool 20. Additionally, or alternatively, the control can be performed to offset or reduce the estimated deflection.

For example, at 214, the controller(s) can adjust a tool path TP of the tool 20. One or more segments of the tool path TP can be dynamically modified to reduce force applied to the tool 20 by the anatomy A. For example, the tool path TP can be partially shifted to reduce the overlap between the radius of a spherical bur and the anatomy A. In other examples, the number of passes (back and forth path oscillations) of the tool path TP can be dynamically adjusted to be more frequent. The depth of the tool path TP can also be modified. For example, arm deflection typically causes undercutting. Therefore, in the case of arm deflection, the depth of the tool path can be increased to compensate for the undercutting. Although this may cause more force to be applied to the tool 20, the result would be an improvement in cutting accuracy. The extent of the tool path TP modifications can correspond to the magnitude of estimated deflection. If the estimated deflection no longer persists, the tool path TP can be returned to the default or last setting.

At 216, the controller(s) can modify a feed rate of the tool 20. The feed rate is the velocity or speed at which the tool 20 traverses the tool path TP. Based on the estimated deflection, the tool 20 feed rate can be slowed to reduce the interaction force applied to the tool 20 by the anatomy A. The slowing can be gradual or immediate. The extent of the feed rate decrease can correspond to the magnitude of estimated deflection. If the estimated deflection no longer persists, the feed rate can be resumed to the default or last setting.

At 218, the controller(s) can modify a pose of the tool 20. The pose can include the TCP and the shaft/body of the tool 20. The tool 20 pose can be modified to increase tool accuracy and/or offset or reduce the estimated deflection. For example, the position of the TCP can be adjusted to account for the estimated deflection values (x, y, z) outputted by the machine learning model ML. In other examples, the orientation of the tool 20 can be adjusted to reduce interaction of the tool 20 with the anatomy A. In some cases, if the tool shaft is colliding with the anatomy A, the tool shaft orientation can be adjusted. There might be some cases to avoid arm deflection as an appropriate action. The pose change can be gradual or immediate. The extent of the pose change can correspond to the magnitude of estimated deflection. If the estimated deflection no longer persists, the pose can return to the standard commanded/controlled pose of the tool 20.

At 220, the controller(s) can actively move the tool 20 away from the anatomy. This technique may be done as a precautionary measure to provide a pause for the operator to re-evaluate the operating conditions of the tool 20 or to alleviate deflections. The tool 20 can be moved away from the anatomy A by a minor distance such that the tool 20 can readily re-engage its movement if re-activated. In other examples, the tool 20 can be significantly moved, e.g., to a resting position spaced far away from the anatomy A. This pull-away action can be gradual or immediate and the timing or execution speed of the pull-away can correspond to the magnitude of estimated deflection. If the estimated deflection no longer persists, the tool 20 can be returned to its last known pose or position on the tool path TP. The return can be automatically or manually performed.

At 222, the controller(s) can modify a cutting speed and/or direction of the tool 20. The cutting speed is the rotational speed (e.g., RPM) at which the energy applicator, such as cutting bur, rotates. The cutting direction is the rotational direction at which the energy applicator rotates (e.g., clockwise, or counterclockwise). Based on the estimated deflection, the cutting speed and/or direction can be modified to reduce the interaction force applied to the tool 20 by the anatomy A. For example, the cutting speed can be reduced gradually or immediately to decrease contact forces. For certain segments of the tool path TP, the cutting direction can be changed to utilize climb milling vs. conventional milling. The timing and extent of the modifications to the cutting speed and/or direction can correspond to the magnitude of estimated deflection. If the estimated deflection no longer persists, the cutting speed and/or direction can be resumed to the default or last setting.

At 224, the controller(s) can actively stop the tool 20. Stopping can involve stopping all energy applied to the tool 20, including zeroing out the feed rate and cutting speed. Similar to pulling the tool away, this technique may be done as a precautionary measure to provide a pause for the operator to re-evaluate the operating conditions of the tool 20 or to alleviate the estimated deflection. The tool 20 can be stopped for any suitable amount of time. Stoppage can be gradual or immediate and the timing or execution speed of the stoppage can correspond to the magnitude of estimated deflection. If the estimated deflection no longer persists, power to the tool 20 can be returned automatically or manually in response to user-acknowledgement.

At 226, the controller(s) can generate or modify a virtual haptic setting of the manipulator 14 and/or tool 20 based on the estimated deflection. For example, the controller(s) can dynamically generate a virtual object VO or boundary 71 based on the estimated deflection. The virtual object or boundary can be like any of those described above. In one example, the virtual boundary or object can be generated based on the location and/or magnitude of the estimated deflection. The virtual object or boundary can limit movement of the tool 20 and/or robotic arm to prevent deflections, e.g., during certain times or in certain regions that are susceptible to high deflection. In one example, the virtual object may be a volume that the tool 20 is constrained to stay within or keep out. In another example, the virtual object may be a mesh of variable constraint (stiffness/damping) parameters that are determined based on the estimated deflection over the surface of the bone. Such implementations can be like those described in U.S. Patent No. 11,986,260, entitled "Robotic surgical system and methods utilizing virtual boundaries with variable constraint parameters", the entire contents of which are hereby incorporated by reference. In yet another example, the virtual boundaries can be layered based on criticality or proximity to target surfaces, such as that described in U.S. Patent No. 10,098,704, entitled "System and method for manipulating an anatomy", the entire contents of which are hereby incorporated by reference. The virtual object or boundary can be enforced for any suitable amount of time. If the estimated deflection no longer persists, the virtual object or boundary can be disabled automatically or manually in response to user-acknowledgement. Any of example steps 214-226 can be executed simultaneously, and in combination.

### C. Total Arm Deflection Estimation

According to another implementation, as shown in FIG. 7, a technique/method 300 implemented by the controller(s) is provided for estimating the total deflection of the manipulator 14 responsive to the various forces described herein, such as the interaction force, user-applied force, and disturbance force. Again, this estimated total deflection is a non-geometric error. Some steps described below can be performed in the manner described with respect to the steps of technique/method 200, and therefore, the description of such steps has been reduced for readability purposes. Additionally, not all steps shown in FIG. 7 are required and some steps may be optional or not applicable given the conditions.

At 302, the controller(s) obtain the pose of the surgical tool 20 based on kinematic data from the manipulator 14. At 304, if applicable, the controller(s) obtain the interaction force applied to the surgical tool 20 responsive to interaction of the surgical tool 20 with the anatomy or patient tissue, in the manner described above.

At 306, if applicable, the controller(s) obtain the user-applied force on the surgical tool 20 responsive to user applying an external force to move the tool 20. The user-applied force is derived based on measurements from the sensing system. As described above, the sensing system can obtain such measurements from various sources. In one implementation, the measurements are provided by the force/torque sensor S. The user-applied force may be defined relative to the TCP of the tool 20 and defined by three axial force components, or force/torque components in six degrees-of-freedom. The user-applied force can be mapped to the robot base 16. The user-applied force can be obtained at a time step, T, e.g., the same time the pose of the tool 20 is obtained at step 302 and/or the interaction force is obtained at 304. The user-applied force can be obtained at discrete times or monitored over a period of time. The period of time can be triggered in response to certain conditions, such as initial detection of the user-applied force.

At 306, if applicable, the controller(s) obtain the disturbance force on the one or more links 18 of the manipulator 14 responsive to some external force applied to the robotic arm. The disturbance force is derived based on measurements from the sensing system. In one implementation, the measurements are provided by current sensors from the joint J actuators/motors. However, other sensing techniques are contemplated. The disturbance force may be defined relative to the TCP of the tool 20 and defined by three axial force components, or force/torque components in six degrees-of-freedom. The disturbance force can be mapped to the robot base 16. The disturbance force can be obtained at a time step, T, e.g., the same time the pose of the tool 20 is obtained at step 302, the interaction force is obtained at 304, and/or the user-applied force is obtained at step 306. Again, the disturbance force can be obtained at discrete times or monitored over a period of time. The period of time can be triggered in response to certain conditions, such as initial detection of the disturbance force.

At 310, the controller(s) input the pose of the surgical tool 20, and where applicable, the interaction force, user-applied force, and/or disturbance force to a machine learning model ML'. The machine learning model ML' can utilize these various inputs for "on the fly" non-geometric error compensation during surgery. The machine learning model can be like any of the examples described above, such as, but not limited to, a deep learning model that includes at least two hidden layers. The machine learning model ML' takes values from the pose of the surgical tool 20 and the various forces (if applicable) at an input layer having an appropriate number of input nodes/neurons for the number of input values. The hidden layers learn how much total robotic arm deflection occurs given the presence of the various forces. The machine learning model ML' can be trained or pre-trained in the manners described above. The output of the machine learning model ML' may be an output layer comprising any suitable number of nodes, such as three nodes each predicting the total estimated deflection of the manipulator 14 for one axial degree of freedom (x, y, z). The output can be characterized as the x, y, z error displacement of the TCP position relative to its commanded/controlled position at the time step T, the time at which the applicable forces and tool pose were acquired. The output can also predict the deflection in the orientation (pitch, roll, yaw) of the tool 20 due to non-geometric errors.

At 312, the controller(s) utilize the output values of the machine learning model ML' to estimate the total deflection of the manipulator 14 and/or surgical tool 20. Deflection and/or duration thresholds can be used to filter out negligible deflection estimates. The controller(s) can utilize the machine learning model ML' to output the estimated total deflection for any number of time-steps during runtime of the manipulator 14. The controller(s) can trigger the deflection monitoring mode to estimate the total deflection and may do so based on any of the triggering conditions described above.

Using the estimated total deflection, the controller(s) can perform the various tasks described above, which can be performed for a subsequent time step T + 1 and can persist thereafter. Any of the tasks described herein can be combined in part, or in whole, and can be executed simultaneously or at different times.

In one example, at 314, the controller(s) can generate a warning/notification related to the estimated total deflection. Additionally, or alternatively, at 316, the controller(s) can control the manipulator 14 and/or tool 20 based on the estimated total deflection. The examples for how the controller(s) can control the manipulator 14 based on the estimated deflection can be like those described above. For example, at 318, the controller(s) can adjust the tool path TP of the tool 20. At 320, the controller(s) can modify the feed rate of the tool 20. At 322, the controller(s) can modify a pose of the tool 20. At 324, the controller(s) can actively move the tool 20 away from the anatomy. At 326, the controller(s) can modify the cutting speed and/or direction of the tool 20. At 328, the controller(s) can stop all power to the tool 20 or disable all tool operations. At 330, the controller(s) can generate or modify a virtual haptic setting of the manipulator 14 and/or tool 20 based on the estimated deflection, e.g., to guide/constrain the tool to improve accuracy. Any of example steps 318-330 can be executed simultaneously, and in combination.

### III. Techniques for Characterization of Tool-Anatomy Interaction Based On Estimated Arm Deflection

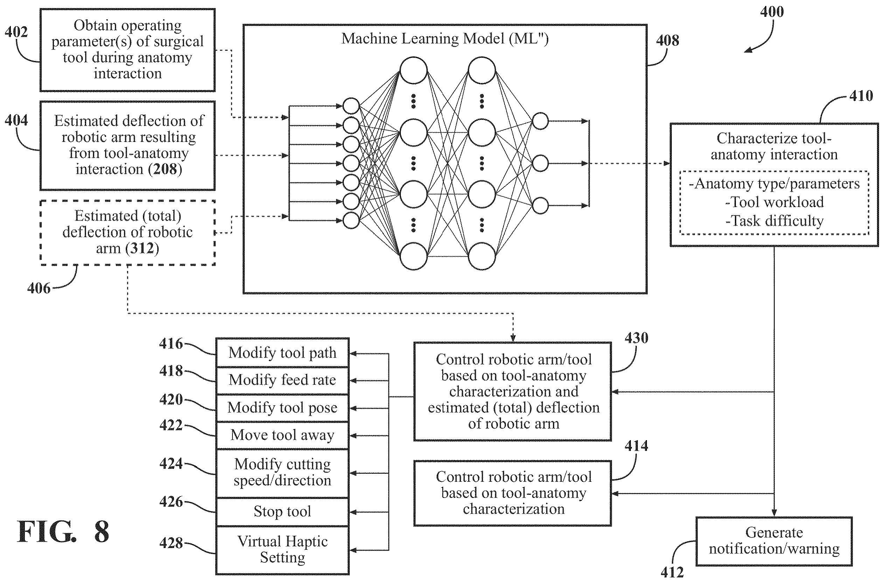

Referring to Figures 8-10, described herein are various techniques for characterizing the tool-anatomy interaction, for example, using the estimated deflection output(s), described above, as an input. In turn, by using the estimated deflection, the techniques described herein advantageously provide a higher order prediction of the tool-anatomy interaction beyond conventional feedback means responsive to tool sensors. For example, through these techniques, the controller(s) can make determinations about the nature of the tissue being interacted with, the workload of the tool 20, and/or the difficulty of task being performed by the tool 20. The controller(s) then can dynamically react to such determinations in real-time. As a result, the accuracy of the tool 20 in cutting/resecting the anatomy will be increased thereby improving clinical outcomes and reducing surgical operating time needed to correct cutting inaccuracies resulting from sub-optimal tool-anatomy interactions. The techniques are able to characterize the tool-anatomy interaction based on estimated robotic arm deflection. In turn, the techniques described herein can characterize the interaction on the robotic arm side, thereby avoiding total reliance on measurements at the interaction site. For example, if the tool 20 were to stall, the techniques can still estimate the robotic arm deflection, whereas other techniques that are reliant on tool operation cannot.

Once again, the techniques described herein can be implemented by any of the described control system(s), controller(s), and/or processors including but not limited to the control system 60, manipulator controller 26, tool controller 21, navigation controller 36, or any combination thereof. For simplicity, the one or more devices will be referred to herein as "the controller(s)." In some instances, any of the data described herein can be sourced from or transmitted to a remote server.

According to one implementation, as shown in FIG. 8, a technique/method 400 implemented by the controller(s) is provided for characterizing the tool-anatomy interaction. Some steps described below can be performed in the manner described with respect to the previously described steps of technique/method 200 or 300, and therefore, the description of such steps has been reduced for readability purposes. Additionally, not all steps shown in FIG. 8 are required and some steps may be optional or not applicable given the conditions.

At 402, the controller(s) obtain one or more operating parameters of the surgical tool 20 during an interaction of the surgical tool 20 with the anatomy A. Example operating parameters of the surgical tool 20, can include, but are not limited to any one or more of the following tool parameters: displacement, velocity; acceleration; a motor current, torque/force, pressure, feed rate, cutting speed, cutting direction, temperature, or the like. The operation parameter(s) can be obtained by the sensing system component(s). Additionally, the navigation system 32 can provide operating parameters based on tracked data. The controller(s) can computer average values for any of the parameters, such as an average velocity or average motor current. Any type of averaging filter can be utilized, such as a moving average filter. The operating parameters can relate to any component of the tool 20, such as the energy applicator, TCP, tool shaft, handpiece, motors/actuators, controllers/PCB, visual indicators, triggers/buttons, or the like. The operating parameter(s) can be obtained at any time during the procedure. Any of the operating parameters can be obtained discretely or over monitored over a period of time. In one example, the operating parameter(s) of the tool 20 can be obtained at a time step, T. In some cases, the controller(s) may request the operating parameter(s) in response to detection of certain conditions, such as detecting the tool 20 interacting with the anatomy A or approaching the anatomy A.

The controller(s) also obtain estimate(s) of the robotic arm deflection, as described above. For example, at 404, the controller(s) can obtain the estimated deflection of the robotic arm resulting from the interaction of the surgical tool with the anatomy A. This estimated deflection can be generated based on the output of step 208, in FIG. 6. Additionally, or alternatively, at 406, the controller(s) optionally obtain the total estimated deflection of the robotic arm, which was generated based on the output of step 312, in FIG. 7. As described, the total estimated deflection may be based in part on the estimated deflection based on tool-anatomy interaction. In either case, the various implementations for estimating the robotic arm deflection, which were described above, are fully incorporated by reference in this section and are not repeated for simplicity.

At 408, the controller(s) input the obtained operating parameter(s) of the tool 20, and the estimated (tool-anatomy and/or total) deflection of the robotic arm to a machine learning model ML". The machine learning model ML" can utilize these various inputs for making "on the fly" characterizations of tool-anatomy interactions during surgery. The machine learning model ML" can be like any of the examples described above, such as, but not limited to, a deep learning model that includes at least two hidden layers. In some cases, the controller(s) utilize at least two machine learning models, e.g., one model ML/ML' for estimating arm deflection and the second model ML" for characterizing tool-anatomy interaction. In other instances, any of the describe machine learning models ML, ML,' ML" can be incorporated into a single trained machine learning model, e.g., that can estimate arm deflection and characterize tool-anatomy interaction.

To characterize tool-anatomy interaction, the machine learning model ML" can be trained or pre-trained in various manners. The hidden layers can be trained online or offline on predetermined values of tool operating parameters and estimated deflections. For example, the machine learning model ML" can be trained on a manipulator 14 that is already fully calibrated using level-1 and level-2 kinematic (geometric) calibration, and optionally, and that further incorporates the estimated deflection compensation techniques described above. In some cases, during training, the manipulator 14 can be moved to enable the tool 20 to interact with a physical anatomy model with various applied forces and in various poses to mimic tool interaction during surgery. Additionally, or alternatively, the hidden layers can be trained online based on values of tool operating parameters and estimated deflections acquired in real-time during surgery. Training can include tissue parameter/type labeling for the specific patient or for a statistical population of patients. The training can be performed off-line autonomously with multiple tissue samples (real or/and phantoms) with different elastic Moduli while measuring the tool(arm) parameters as well as the arm deflection. The machine learning model can be also trained for the bone density by analyzing historical data for multiple cases with different bone densities, extracting the average measured force during bone resection, the arm pose for the particular bone resection that can be fed into the machine learning model ML (e.g., FIG. 6) to estimate the arm deflection for the given case. The estimated arm deflection and the arm parameter can be then used to train the machine learning model ML" (e.g., FIG. 8) as the bone density for that particular case is also known (supervised learning)

The machine learning model ML" takes values from the operating parameter(s) of tool 20 and the estimated (tool-anatomy and/or total) deflection of the robotic arm at an input layer having an appropriate number of input nodes/neurons for the number of input values. The hidden layers learn how to characterize the tool-anatomy interaction given the presence of various estimated robotic arm deflections. The output of the machine learning model ML" may be an output layer comprising any suitable number of nodes, depending on the nature/type of the characterization. The output may comprise text, measurements, images, or any suitable output form appropriate to characterize tool-anatomy interaction.

At 410, based on the output of the machine learning model ML", the controller(s) obtain a characterization and/or classification of the tool-anatomy interaction. The tool-anatomy interaction can be characterized in various manners. In one example, the machine learning model ML" characterizes the tool-anatomy interaction by characterizing a type of anatomy A. For example, the type of anatomy A can be characterized by the type of tissue of the anatomy A, such as bone, soft tissue, ligament, tendon, cartilage, osteophyte, cancellous tissue, cortical tissue, or the like. Additionally, the type of anatomy A can be characterized by the label/name of the body part, such as femur, tibia, acetabulum, scapula, glenoid, etc. For revision surgery, where there is an existing primary implant in the bone, the machine learning model ML" may be able to identify other materials or objects, such as the presence of the implant, or characterize a feature or type of the implant, e.g., metal implant, articular implant, screw, etc.

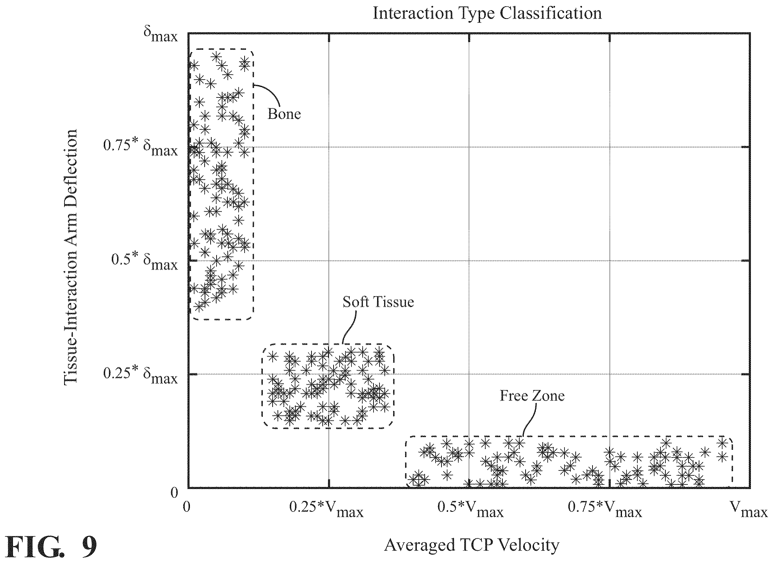

To illustrate further, FIG. 9 provides a chart showing an example classification outputted by the machine learning model ML". Here, the operating parameters of the tool 20 include an average velocity of the TCP and the estimated deflection is based on the tool-anatomy interaction. Upon training, the machine learning model ML" learned that the tissue-interaction arm deflection values exhibit a pattern wherein values become grouped into either a bone region, a soft tissue region or a free zone region. The free zone region indicates that the tool 20 is moving in space, but not directly interacting with the anatomy A. For any given time step, T, the machine learning model ML" can predict whether the tool 20 is interacting with bone, soft tissue, or air. Similar machine learning recognition can be realized using other values to determine the various other classifications described above.

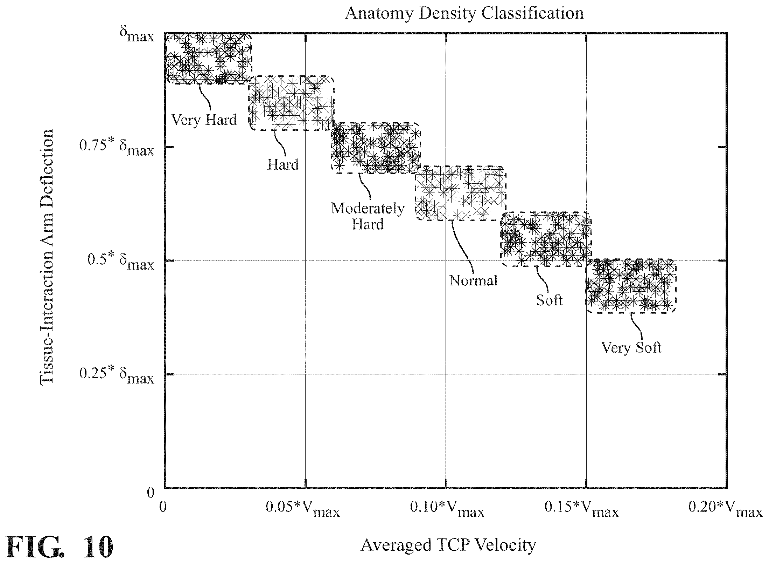

Continuing at 410, the tool-anatomy interaction can also be characterized by classifying parameters/features of the anatomy A. By using the estimated arm deflection and tool operating parameter(s), the machine learning model ML" can make predictions regarding the anatomy density, hardness, softness, etc. To illustrate further, FIG. 10 provides another chart showing an example classification outputted by the machine learning model ML". Here, the operating parameters of the tool 20 once again include an average velocity of the TCP and the estimated deflection is based on the tool-anatomy interaction. Upon training, the machine learning model ML" learned that the tissue-interaction arm deflection values exhibit a pattern wherein values become grouped into six regions of anatomy (tissue) density, ranging from very soft to vary hard. The label of these groupings is provided merely as an example. The groupings can have any label or need not be labeled at all. Additionally, the classification groupings can be labeled by values or value ranges of density/hardness. The very soft region may indicate that the tool 20 is interacting with a soft tissue and the very hard region may indicate that the tool 20 is interacting with a particularly hard material, such as dense cortical femur bone. For any given time step, T, the machine learning model ML" can predict the parameters of the anatomy. Similar machine learning recognition can be realized using other values to determine the various other classifications described above, or combinations thereof. Aside from average TCP velocity, any of the various operating parameter(s) described herein can be utilized to estimate parameters of the anatomy.

The techniques described herein are quite advantageous for characterizing bone density. Bone density is associated with the arm deflection. Many conventional approaches in clinical applications rely on tissue deformation and force applied to the tissue to characterize the tissue. When it comes to bony tissue, there is minimal, if any, tissue deformation. Instead, the robotic arm deflection typically occurs because external loads are unable to deform the bony tissue. Therefore, the proposed techniques here would be more efficient to estimate bone density compared to the prior methods.

With continued reference to 410, the tool-anatomy interaction can also be characterized by a workload or task difficulty. By using the estimated arm deflection and tool operating parameter(s), the machine learning model ML" can make predictions regarding how hard the tool 20 is working during operation and/or how hard the task is that is being performed by the tool 20. For example, the machine learning model ML" can be trained to combine classifying task difficulty with classifying anatomy type/parameters. In other examples, the machine learning model ML" can estimate workload or task difficulty by predicting energy transfer or work as a function of force and displacement. For example, groupings can be classified similar to those groupings shown in FIG. 10, wherein the machine learning model ML" can predict that the tool 20 is interacting with a soft tissue and hence does not exhibit much difficulty in performing its task (e.g., cutting/probing/etc.). The machine learning model ML" can predict that the tool 20 is interacting with a particularly hard material, such as dense cortical femur bone, and hence, is exhibiting much work in performing its task. For any given time step, T, the machine learning model ML" can predict workload or task difficulty of the tool 20. Any of the various operating parameter(s) described herein can be utilized to estimate workload or task difficulty.

Having made the appropriate tool-anatomy interaction characterization/classification, the controller(s) can then perform various tasks. Any of these tasks can be performed for a subsequent time step T +1 and can persist thereafter. Any of the tasks described herein can be combined in part, or in whole, and can be executed simultaneously or at different times.

In one example, at 412, the controller(s) can generate a warning/notification related to the tool-anatomy interaction characterization. For instance, the warning/notification can be immediately presented on the display 38 of the navigation system 32 to communicate to the operator a message, such as "Excessive Tool Workload". In another example, after characterizing the tissue and workload, the controller(s) can display a message and/or sound an alert to the user to prevent tissue damage. Additionally, or alternatively, any appropriate warning can be conveyed through the manipulator 14, e.g., using vibratory feedback to the user through the surgical tool 20 or by illuminating of an indicator on the manipulator 14 a certain color, such as red. Other types of audio/visual notifications can also be communicated to the operator.

Additionally, or alternatively, at 414, the controller(s) can control the manipulator 14 and/or tool 20 based on the tool-anatomy interaction characterization. For example, the controller(s) can control the manipulator 14 and/or tool 20 to dynamically adapt to changes in tissue/material type or to alleviate task difficulty/workload. The examples for how the controller(s) can control the manipulator 14 based on the tool-anatomy interaction characterization can be like those described above. For example, at 416, the controller(s) can adjust the tool path TP of the tool 20. At 418, the controller(s) can modify the feed rate of the tool 20. At 420, the controller(s) can modify a pose of the tool 20. At 422, the controller(s) can actively move the tool 20 away from the anatomy. At 424, the controller(s) can modify the cutting speed and/or direction of the tool 20. At 426, the controller(s) can stop all power to the tool 20 or disable all tool operations. At 428, the controller(s) can generate or modify a virtual haptic setting of the manipulator 14 and/or tool 20 based on the estimated deflection, e.g., to guide/constrain the tool to improve accuracy. Any of example steps 416-428 can be executed simultaneously, and in combination.

In one example, the machine learning model ML" predicts a level of task difficulty at a time step T and the controller(s) in turn calculates a reduced feed rate of the tool 20 to avoid clogging of the tool 20 during cutting. The reduced feed rate is communicated to the tool controller 21 to dynamically slow the tool 20 at T+1.

In another example, at 430, the total estimated arm deflection (312, 406) is utilized (without being inputted into the machine learning model ML"). Here, the controller(s) can control the manipulator 14 and/or tool 20 based on the tool-anatomy interaction characterization and the total estimated arm deflection. For instance, the tool path generator 68 can utilize a task difficulty classification (or reduced feed rate) as well as the total estimated deflection to adjust the tool path TP in real time or perform any of the tasks 416-428 described above.

In other examples, the manipulator 14 can be coupled to supplemental systems that can be controlled based on the tool-anatomy interaction output of 410. For example, an irrigation or aspirator system can be used coupled to the manipulator 14 and/or tool 20 to irrigate and/or aspirate the surgical site, which can affect the tool's interaction with the anatomy. Based on the characterized tool-anatomy interaction at 410, the controller(s) can control the irrigation or aspirator system to modify the magnitude/frequency of irrigation/aspiration. For example, if the machine learning model ML" predicts that the tool 20 is experiencing heavy workload, irrigation can be increased to reduce the tool workload. In other examples, the navigation system 34 can be controlled based on the output of 410. For example, tracking modality/performance can be modified depending on the detected type/parameters of the tissue. If the tool is a camera or is equipped with an illumination device, the camera or illumination device can be controlled based on the output of 410. Other examples are contemplated.

Several embodiments have been described in the foregoing description. However, the embodiments discussed herein are not intended to be exhaustive or limit the invention to any particular form. The terminology, which has been utilized, is intended to be in the nature of words of description rather than of limitation. Many modifications and variations are possible in light of the above teachings and the invention may be practiced otherwise than as specifically described.

The present disclosure also comprises the following clauses, with specific features laid out in dependent clauses, that may specifically be implemented as described in greater detail with reference to the configurations and drawings above.

### CLAUSES

C1. A surgical system comprising: a robotic arm comprising a plurality of links and joints; a surgical tool supported and moveable by the robotic arm and being configured to interact with an anatomy; one or more controllers coupled to the robotic arm and being configured to: obtain an operating parameter of the surgical tool during an interaction of the surgical tool with the anatomy; estimate a deflection of the robotic arm resulting from the interaction of the surgical tool with the anatomy; input the operating parameter of the surgical tool and the estimated deflection into to a machine learning model; and characterize the interaction of the surgical tool with the anatomy based on an output of the machine learning model.
C2. The surgical system of clause C1, wherein the one or more controllers characterize the interaction by characterizing a type of the anatomy.
C3. The surgical system of clause C2, wherein the one or more controllers characterize the type of the anatomy as bone or soft tissue.
C4. The surgical system of any preceding clause, wherein the one or more controllers characterize the interaction by characterizing a workload of the surgical tool.
C5. The surgical system of clause C4, wherein the one or more controllers are configured to modify a feed rate of the surgical tool in response to characterizing the workload of the surgical tool.
C6. The surgical system of any preceding clause, wherein the one or more controllers are configured to control the robotic arm and/or the surgical tool based on the characterized interaction.
C7. The surgical system of clause C6, wherein the one or more controllers control the robotic arm and/or the surgical tool based on the characterized interaction by being configured to perform one or more of: modify a tool path of the surgical tool; modify a feed rate of the surgical tool; modify a cutting speed of the surgical tool; adjust a commanded pose of the surgical tool; halt movement of the surgical tool; move the surgical tool away from the anatomy; and/or generate or modify a virtual haptic setting for the surgical tool.
C8. The surgical system of any preceding clause, wherein the one or more controllers are configured to generate a notification or warning based on the characterized interaction.
C9. The surgical system of any preceding clause, comprising: a sensing system configured to detect an interaction force applied to the surgical tool based on interaction of the surgical tool with the anatomy; and the one or more controllers being configured to: obtain, based on kinematic data from the robotic arm, a pose of the surgical tool; obtain, from the sensing system, the interaction force; input the pose of the surgical tool and the interaction force to a second machine learning model; and estimate the deflection of the robotic arm resulting from the interaction of the surgical tool with the anatomy based on an output of the second machine learning model.
C10. The surgical system of any preceding clause, comprising: a sensing system configured to detect: an interaction force applied to the surgical tool based on interaction of the surgical tool with the anatomy; a disturbance force applied to one or more of the links; and a user-applied force on the surgical tool; and the one or more controllers being configured to: obtain, based on kinematic data from the robotic arm, a pose of the surgical tool; obtain, from the sensing system, the interaction force, the disturbance force, and the user-applied force; input, to a second machine learning model, the pose of the surgical tool, the interaction force, the disturbance force, and the user-applied force; and estimate a total deflection of the robotic arm based on an output of the second machine learning model.
C11. The surgical system of clause C10, wherein the one or more controllers control the robotic arm and/or the surgical tool based on the characterized interaction and based on the total deflection of the robotic arm by being configured to perform one or more of: modify a tool path of the surgical tool; modify a feed rate of the surgical tool; modify a cutting speed of the surgical tool; adjust a commanded pose of the surgical tool; halt movement of the surgical tool; move the surgical tool away from the anatomy; and/or generate or modify a virtual haptic setting for the surgical tool.
C12. The surgical system of any preceding clause, wherein: the surgical tool comprises a tool center point (TCP); and the one or more controllers obtain the operating parameter of the surgical tool during the interaction of the surgical tool with the anatomy by being configured to obtain the operating parameter of the TCP during the interaction.
C13. The surgical system of any preceding clause, wherein the operating parameter of the surgical tool comprises one or more of: a velocity of the surgical tool; an average velocity of the surgical tool; an acceleration of the surgical tool; displacement of the surgical tool; a motor current of the surgical tool.
C14. The surgical system of any preceding clause, wherein the robotic arm is configured to operate in a manual mode wherein the robotic arm is configured to move the surgical tool in response to forces applied to the surgical tool by a user.
C15. The surgical system of any preceding clause, wherein the robotic arm is configured to operate in an automated mode wherein the robotic arm is configured to move the surgical tool autonomously along a tool path.
C16. A surgical system comprising: a robotic arm comprising a plurality of links and joints; a surgical tool supported and moveable by the robotic arm and being configured to interact with an anatomy; one or more controllers coupled to the robotic arm and being configured to: estimate a deflection of the robotic arm resulting from the interaction of the surgical tool with the anatomy; input the estimated deflection into to a machine learning model; and characterize the interaction of the surgical tool with the anatomy based on an output of the machine learning model.
C17. A method of operating a surgical system that includes a robotic arm comprising a plurality of links and joints, a surgical tool supported and moveable by the robotic arm and being configured to interact with an anatomy, and one or more controllers coupled to the robotic arm, the method comprising the one or more controllers performing the following: obtaining an operating parameter of the surgical tool during an interaction of the surgical tool with the anatomy; estimating a deflection of the robotic arm resulting from the interaction of the surgical tool with the anatomy; inputting the operating parameter of the surgical tool and the estimated deflection into to a machine learning model; and characterizing the interaction of the surgical tool with the anatomy based on an output of the machine learning model.

## Claims

1. A surgical system (10) comprising:
a robotic arm (14) comprising a plurality of links (18) and joints (J);
a surgical tool (20) supported and moveable by the robotic arm (14) and being configured to interact with an anatomy (A);
a sensing system (14, 19, 20, 32, J, S) configured to detect an interaction force applied to the surgical tool (20) based on an interaction of the surgical tool (20) with the anatomy (A); and
one or more controllers (60, 26, 21, 36) coupled to the robotic arm (14) and the sensing system (14, 19, 20, 32, J, S) and being configured to:
obtain, based on kinematic data from the robotic arm (14), a pose of the surgical tool (20);
obtain, from the sensing system (14, 19, 20, 32, J, S), the interaction force;
input the pose of the surgical tool (20) and the interaction force to a machine learning model (ML, ML', ML");
estimate a deflection of the robotic arm (14) based on an output of the machine learning model (ML, ML', ML"); and
control the robotic arm (14) and/or the surgical tool (20) based on the estimated deflection.

2. The surgical system (10) of claim 1, wherein:
the sensing system (14, 19, 20, 32, J, S) is further configured to detect a user-applied force on the surgical tool (20); and
the one or more controllers (60, 26, 21, 36) are further configured to:
obtain, from the sensing system (14, 19, 20, 32, J, S), the user-applied force;
input the user-applied force to the machine learning model (ML, ML', ML");
estimate the deflection of the robotic arm (14) based on the output of the machine learning model (ML, ML', ML"); and
control the robotic arm (14) and/or the surgical tool (20) based on the estimated deflection.

3. The surgical system (10) of any preceding claim, wherein:
the sensing system (14, 19, 20, 32, J, S) is further configured to detect a disturbance force applied to one or more of the links (18) of the robotic arm (14); and
the one or more controllers (60, 26, 21, 36) are further configured to:
obtain, from the sensing system (14, 19, 20, 32, J, S), the disturbance force;
input the disturbance force to the machine learning model (ML, ML', ML");
estimate the deflection of the robotic arm (14) based on the output of the machine learning model (ML, ML', ML"); and
control the robotic arm (14) and/or the surgical tool (20) based on the estimated deflection.

4. The surgical system (10) of any preceding claim, wherein:
the sensing system (14, 19, 20, 32, J, S) is further configured to detect:
a disturbance force applied to one or more of the links (18) of the robotic arm (14); and
a user-applied force on the surgical tool (20); and
the one or more controllers (60, 26, 21, 36) are further configured to:
obtain, from the sensing system (14, 19, 20, 32, J, S), the disturbance force;
obtain, from the sensing system (14, 19, 20, 32, J, S), the user-applied force;
input the disturbance force and the user-applied force to the machine learning model (ML, ML', ML");
estimate the deflection of the robotic arm (14) based on the output of the machine learning model (ML, ML', ML"); and
control the robotic arm (14) and/or the surgical tool (20) based on the estimated deflection.

5. The surgical system (10) of any preceding claim, wherein the one or more controllers (60, 26, 21, 36) are configured to:
control the robotic arm (14) to move the surgical tool (20) along a tool path (TP) to interact with the anatomy (A); and
control the robotic arm (14) and/or the surgical tool (20) based on the estimated deflection by being configured to modify the tool path (TP).

6. The surgical system (10) of any preceding claim, wherein the one or more controllers (60, 26, 21, 36) are configured to:
control the robotic arm (14) to move the surgical tool (20) at a feed rate to interact with the anatomy (A); and
control the robotic arm (14) and/or the surgical tool (20) based on the estimated deflection by being configured to modify the feed rate.

7. The surgical system (10) of any preceding claim, wherein the one or more controllers (60, 26, 21, 36) are configured to:
control the robotic arm (14) to move the surgical tool (20) to a commanded pose; and
control the robotic arm (14) and/or the surgical tool (20) based on the estimated deflection by being configured to adjust the commanded pose.

8. The surgical system (10) of any preceding claim, wherein the one or more controllers (60, 26, 21, 36) are configured to:
obtain an operating parameter of the surgical tool (20);
input the operating parameter of the surgical tool (20) and the estimated deflection into a second machine learning model (ML"); and
characterize the interaction of the surgical tool (20) with the anatomy (A) based on an output of the second machine learning model (ML").

9. The surgical system (10) of claim 8, wherein the one or more controllers (60, 26, 21, 36) characterize the interaction by characterizing a type of the anatomy (A) and/or a workload of the surgical tool (20).

10. The surgical system (10) of any one of claims 8 to 9, wherein the one or more controllers (60, 26, 21, 36) control the robotic arm (14) and/or the surgical tool (20) based on the characterized interaction by being configured to perform one or more of:
modify a tool path (TP) of the surgical tool (20);
modify a feed rate of the surgical tool (20);
modify a cutting speed of the surgical tool (20);
adjust a commanded pose of the surgical tool (20);
halt movement of the surgical tool (20);
move the surgical tool (20) away from the anatomy (A); and/or
generate or modify a virtual haptic setting for the surgical tool (20).

11. The surgical system (10) of any preceding claim, wherein:
the surgical tool (20) is configured to cut tissue of the anatomy (A); and
the interaction force is based on the interaction of the surgical tool (20) with the tissue during cutting of the tissue;
and optionally,
wherein the sensing system (14, 19, 20, 32, J, S) comprises at least a force/torque sensor (S) coupled to a distal link of the robotic arm (14).

12. The surgical system (10) of any preceding claim, wherein the estimated deflection is a non-geometric deflection.

13. The surgical system (10) of any preceding claim, wherein the one or more controllers (60, 26, 21, 36) are configured to trigger a deflection monitoring mode in response to detection of a certain condition, and wherein in the deflection monitoring mode the one or more controllers (60, 26, 21, 36) are configured to:
input the pose of the surgical tool (20) and the interaction force to the machine learning model (ML, ML', ML"); and
estimate the deflection of the robotic arm (14) based on the output of the machine learning model (ML, ML', ML").

14. A method of operating the surgical system (10) of any preceding claim.

15. A non-transitory computer readable medium, or computer program product, comprising instructions, which when executed by one or more processors, are configured to implement operation of the surgical system (10) of any one of claims 1 to 13.
